# EUROPÄISCHE PATENTANMELDUNG

(11) **EP 2 962 679 A1**
(43) Veröffentlichungstag der Anmeldung: **06.01.2016**
(21) Anmeldenummer: 14175031.5
(22) Anmeldetag: 30.06.2014
(51) Int. Cl.: A61K 8/49, A61K 8/97, A61Q 11/00, A23G 3/48, A23G 4/06

(54) **Verwendung von Tetrahydrofuranlignanen**

(71) Anmelder: Symrise AG, 37603 Holzminden (DE)
(72) Erfinder: Ley, Jakob, 37603 Holzminden (DE); Reichelt, Katharina, 37603 Holzminden (DE); Obst, Katja, 37603 Holzminden (DE); Kindel, Günter, 37671 Höxter (DE)
(74) Vertreter: Fabry, Bernd

(57) **Zusammenfassung**

Die Erfindung betrifft Tetrahydrofuranlignane und Extrakte davon, und deren pharmazeutische und kosmetische Verwendung als Kühlwirkstoffe für die Haut und Schleimhaut. Zudem befindet sich die Erfindung auf dem Gebiet Nahrungs- und Lebensmittel, in denen die Tetrahydrofuranlignane und Extrakte davon ebenfalls eingesetzt werden, vorzugsweise in Aromazusammensetzungen und als Geschmacksmodifizierer.

## Beschreibung

### GEBIET DER ERFINDUNG

Die Erfindung betrifft Tetrahydrofuranlignane und Extrakte davon, und deren pharmazeutische und kosmetische Verwendung als Kühlwirkstoffe für die Haut und Schleimhaut. Zudem befindet sich die Erfindung auf dem Gebiet Nahrungs- und Lebensmittel, in denen die Tetrahydrofuranlignane und Extrakte davon ebenfalls eingesetzt werden, vorzugsweise in Aromazusammensetzungen und als Geschmacksmodifizierer.

### STAND DER TECHNIK

Physiologische Kühlwirkstoffe werden regelmäßig eingesetzt, um einen kühlen sensorischen Eindruck auf der Haut bzw. Schleimhaut, zum Beispiel auf der Schleimhaut im Mund-, Nasen- und/oder Rachenraum hervorzurufen, wobei allerdings tatsächlich keine physikalische Abkühlung wie zum Beispiel bei der Verdunstung von Lösungsmitteln stattfindet. Als physiologische Kühlwirkstoffe können sowohl Einzelkomponenten als auch Gemische verwendet werden.

Der bekannteste Kühlwirkstoff ist das natürlich vorkommende L-Menthol, der aber einige Nachteile besitzt, z.B. starker Geruchseindruck, eine hohe Flüchtigkeit und in höheren Konzentrationen einen bitteren und/oder scharfen Eigengeschmack. Der Einsatz von L-Menthol kann somit in bestimmten Aromakompositionen, insbesondere solchen, die nicht in Richtung (Pfeffer)Minz-Aroma gehen, unerwünscht sein.

Es wurden schon früher Untersuchungen durchgeführt, die auf natürlich vorkommende Kühlwirkstoffe ohne Aromaeffekt hinzielen. So wurden z.B. Milchsäureester von Menthol(en) gemäß DE 2 608 226 auch in der Natur identifiziert (Gassenmeier, K., Identification and quantification of L-menthyl lactate in essential oils from Mentha arvensis L. from India and model studies on the formation of L-menthyl lactate during essential oil production. Flavour and Fragrance Journal 2006, 21, (4), 725-730), die zwar eine starke Kühlwirkung haben, deren Hydrolyse in wässrigen Medien aber zu dem stark riechenden Menthol führen können. Menthylmonoester von Disäuren nach US 5,725,865 und US 5,843,466 sind zwar interessante natürlich vorkommende Alternativen, können aber in sensorischen Test nicht die Stärke der vorher beschriebenen Kühlwirkstoffe erreichen. Zudem sind sie als Ester auch empfindlich gegen Hydrolyse. In EP 1,040,765 wurde die Verwendung von Cubebol als Kühlwirkstoff empfohlen, welches aber auch eine relative starke Aromawirkung zeigt. Einige heterocyclische Maillard-Produkte aus Malz zeigten eine interessante Kühlwirkung wie in US 2004 052,828 beschrieben, allerdings ist auch hier ein überaus hoher eigenständiger, nichtkühlender Aromawert hinderlich für eine breite Anwendung. In WO 2006 117,602 wurden bestimmte, aus Hafer isolierte kühlend wirkende Avenanthramide beschrieben. Allerdings sind diese Verbindungen nicht nur in sehr geringen Mengen aus natürlichen Quellen verfügbar sondern auch auf Grund ihrer Zimtsäurestruktur empfindlich gegen Lichteinfluss und können dadurch ihre Wirkung verlieren.

Die Aufgabe der vorliegenden Erfindung hat somit darin bestanden, neue natürlich vorkommende Verbindungen mit physiologischer Kühlwirkung, bereitzustellen. Zudem war es Aufgabe der vorliegenden Erfindung, Verbindungen bereitzustellen, die auch als Geschmacksmodifizierer eingesetzt werden können, um unangenehme Geschmacksnoten im Endprodukt zu maskieren, überdecken oder zu vermindern. Ferner sollen die neu gefundenen Verbindungen für die Zwecke der vorliegenden Erfindung toxikologisch unbedenklich sein, so dass sie breit einsetzbar sind, insbesondere sowohl im Nahrungs- und Lebensmittelbereich, als auch in den pharmazeutischen und kosmetischen Bereichen. Die neuen Verbindungen sollen dabei eine gute (Hydrolyse-) Stabilität aufweisen und insbesondere keinen oder lediglich möglichst einen schwachen Eigengeschmack aufweisen, so dass das positive Geschmacksprofil der Endzubereitung nicht (negativ) beeinflusst wird. Insbesondere sollen die gefundenen neuen Verbindungen für die Zwecke der vorliegenden Erfindung wenig oder gar nicht bitter schmecken, sowie möglichst nicht reizend sein.

### BESCHREIBUNG DER ERFINDUNG

Gegenstand der Erfindung ist die Verwendung von Tetrahydrofuranlignane der allgemeinen Formel (I) oder ein Extrakt davon: 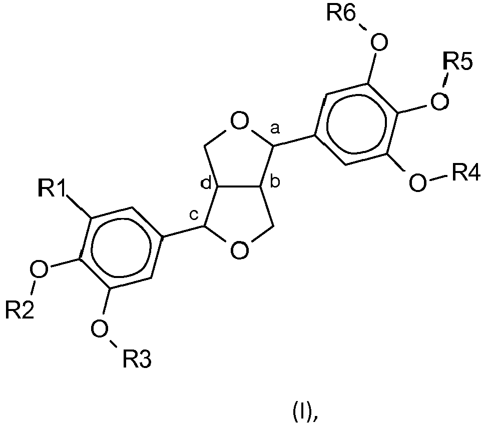
wobei R 1, R2, R3, R4, R5 und R6 unabhängig voneinander ausgewählt sind aus der Gruppe bestehend aus: Wasserstoff, C1-C3-Alkyl,
   und/oder
R2 und R3 jeweils zusammen und/oder
R5 und R6 jeweils zusammen
einen carbocyclischen Ring ausbilden können, ausgewählt aus der Gruppe bestehend aus: -CH2-, -CH2-CH2-, -CH2-CH2-CH2-, -CH2-CH2-CH2-CH2-,
   wobei
die Stereozentren a, b, c und d jeweils unabhängig voneinander eine (R)- oder (S)-Konfiguration aufweisen,
   (i) als Kühlwirkstoffe und / oder
   (ii) als Geschmacksmodifizierer und / oder
   (iii) als Aromastoffe und / oder als geschmacksmodulierende Aromastoffe zur
      a) Maskierung und/oder Verminderung und/oder Unterdrückung und/oder Überdeckung eines unangenehmen Geschmacks, ausgewählt aus bitter, astringend und / oder den Nachgeschmack von Süßstoffen , und / oder
      b) Verstärkung eines angenehmen Geschmacks.

Überraschenderweise wurde gefunden, dass Verbindungen der Formel (I) ein starkes Gefühl der Kälte auf der Haut oder Schleimhaut bewirken, und so einen vorteilhaft kühlenden Effekt auf der Haut oder Schleimhaut vermitteln. Insbesondere im Fall der Schleimhäute sind die der Mund-, Nasen- und Rachenräume gemeint. Es konnten zudem weitere trigeminale Effekte wie Schärfe, Tingling oder Betäubung und in einzelnen Fällen auch mundwässernde Effekte festgestellt werden. Vorteilhafterweise weisen die Verbindungen der Formel (I) keinerlei Nebengeschmack auf, so dass sie keine weiteren Aromawerte besitzen.

Eine weitere überraschende Erkenntnis ist, dass die erfindungsgemäßen Tetrahydrofuranlignane der Formel (I) in Zubereitungen die als negativ empfundene Geschmacks- oder Aromawirkung von schlecht schmeckenden Stoffen, insbesondere bitter oder adstringierend schmeckenden Stoffen, verändern, mindern oder unterdrücken kann und somit die erfindungsgemäßen Tetrahydrofuranlignane der Formel (I) als Geschmacksmodifizierer dienen können.

Unangenehm bzw. schlecht schmeckende Stoffe im Sinne der Erfindung sind Stoffe die adstringierend, trocken, staubig, mehlig, kalkig und/oder metallisch schmecken sowie Stoffe, die einen entsprechenden stark anhaltenden Nachgeschmack aufweisen, wobei der adstringierende Geschmackseindruck dabei oft mit den Geschmackseindrücken trocken, staubig, mehlig, kalkig und/oder metallisch einhergeht.

Daher sind die Verbindungen der Formel (I) insbesondere auch als geschmacksmodulierende Aromastoffe geeignet.

In einer bevorzugten Ausführungsform sind die zur Verwendung eingesetzten Tetrahydrofuranlignane der Formel (I) 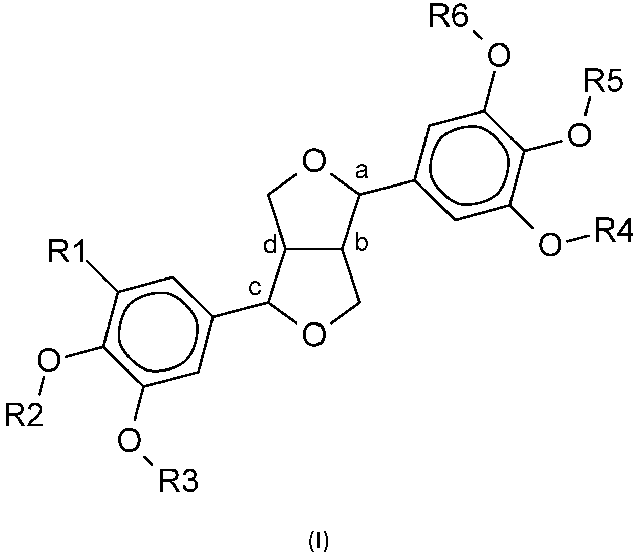 vorzugsweise Verbindungen, wobei
R1 Wasserstoff oder C1-C3-Alkyl ist, und
R2, R3, R4, R5, und R6 unabhängig voneinander ein C1-C3Alkyl darstellen,
   und/oder
R2 und R3 jeweils zusammen und/oder
R5 und R6 jeweils zusammen einen carbocyclischen Ring ausbilden können, ausgewählt aus der Gruppe bestehend aus: -CH2-, -CH2-CH2-, -CH2-CH2-CH2-, -CH2-CH2-CH2-CH2-, wobei
die Stereozentren a, b, c und d jeweils unabhängig voneinander eine(R)- oder (S)-Konfiguration aufweisen.

In einer weiteren bevorzugten Ausführungsform sind die zur Verwendung eingesetzten Tetrahydrofuranlignane der allgemeinen Formel (I), vorzugsweise Verbindungen, wobei
R1 Wasserstoff oder C1-C3-Alkyl ist, und
R2, R3, R4, R5, und R6 unabhängig voneinander ein C1-C3Alkyl darstellen, und/oder
R5 und R6 jeweils zusammen einen carbocyclischen Ring ausbilden können, ausgewählt aus der Gruppe bestehend aus: -CH2-, -CH2-CH2-, -CH2-CH2-CH2-, -CH2-CH2-CH2-CH2-, wobei
die Stereozentren a, b, c und d jeweils unabhängig voneinander eine(R)- oder (S)-Konfiguration aufweisen.

Ferner ist in einer besonders bevorzugten Ausführungsform der vorliegenden Erfindung der carbocyclischer Ring in Formel (I), welcher durch R2 und R3 zusammen und/oder R5 undR6 zusammen ausgebildet wird, vorzugsweise jeweils ein Dioxolanring.

Ebenfalls ist in einer besonders bevorzugten Ausführungsform der vorliegenden Erfindung die C1-C3-Alkygruppe in Formel (I) eine Methylgruppe.
In einer besonders bevorzugten Ausführungsform handelt es sich bei der erfindungsgemäßen Verwendung als Kühlwirkstoff, um eine Verwendung zur physiologischen Kühlung der Haut oder Schleimhaut.

Die Tetrahydrofuranlignane der Formel (I) weisen 4 Stereozentren auf, so dass eine Verbindung im Grunde 24 unterschiedliche Stereoisomere umfassen kann. Demgemäß wird in der vorliegenden Erfindung unter der Formulierung "eine oder mehrere Verbindung der Formel (I)", jedes einzelne Isomer verstanden. So sind beispielsweise unter der allgemeinen Verbindung. Im Sinne der vorliegenden Erfindung wird daher unter eine Mischung von mehreren Verbindungen der Formel (I) demgemäß auch ein Gemisch aus unterschiedlichen Stereoisomeren (Enantiomere und Diastereomere) "derselben Verbindung" verstanden.

6, 6-(3,4,5-Trimethoxyphenyl)-1,3,3a,4,6,6a-hexahydrofuro [3,4-c]furan-3-yl]-4-methoxy-1,3-benzodioxol
alle darunter fallenden einzelne Isomer zu fassen, wie vorzugsweise:
(+)-Episesartemin B (6-[(*3S*,*3aR*,*6R*,*6aR*)-6-(3,4,5-Trimethoxyphenyl)-1,3,3a,4,6,6a-hexahydrofuro [3,4-c]furan-3-yl]-4-methoxy-1,3-benzodioxol;
6-[((*3R,3aS,6S,6aS*)-6-(3,4,5-Trimethoxyphenyl)-1,3,3a,4,6,6a-hexahydrofuro [3,4-c]furan-3-yl]-4-methoxy-1,3-benzodioxol;
(6-[((*3R,3aR,6S,6aS*)-6-(3,4,5-Trimethoxyphenyl)-1,3,3a,4,6,6a-hexahydrofuro [3,4-c]furan-3-yl]-4-methoxy-1,3-benzodioxol; usw.
Oder beispielsweise wird unter der allgemeinen Verbindung
3,6-Bis(3,4,5-trimethoxyphenyl)-1,3,3a,4,6,6a-hexahydrofuro[3,4-c]furan alle darunter fallenden einzelne Isomer zu fassen, wie vorzugsweise:
   (*3R,3aR,6R,6aR*)-3,6-Bis(3,4,5-trimethoxyphenyl)-1,3,3a,4,6,6a-hexahydrofuro [3,4-c]furan;
   (*3S,3aS,6S,6aS*)-3,6-Bis(3,4,5-trimethoxyphenyl)-1,3,3a,4,6,6a-hexahydrofuro [3,4-c]furan;
   (*3R,3aS,6R,6aS*)-3,6-Bis(3,4,5-trimethoxyphenyl)-1,3,3a,4,6,6a-hexahydrofuro [3,4-c]furan; usw.

Es stehen somit 2⁴ -Kombinationsmöglichkeiten für eine Verbindung der Formel (I) zur Auswahl, aus der bis zu 16 Isomere resultieren können, die vorzugsweise folgende Stereozentren aufweisen können:

| | | |
|---|---|---|
| R,R,R,R | R,S,S,S | R,R,S,S |
| S,S,S,S | S,R,R,R | S,S,R,R |
| S,R,S,S | R,R,S,R | R,S,R,S |
| R,S,R,R | S,S,R,S | S,R,S,R |
| S,S,S,R | R,R,R,S | R,S,S,R |

Demgemäß ist in der vorliegenden Erfindung jede der einzelnen Stereoisomere (Enantiomere und Disatereomere) mit eingeschlossen und mitumfasst, auch, wenn im Folgenden nur bestimmte genannt werden.

Besonders zur Anwendung bevorzugt, sind Verbindungen der Formel (I) ausgewählt aus der Gruppe bestehend aus:
6-[((*3S*,*3aR*,*6S*,*6aR*)-3-(3,4-Dimethoxyphenyl)-1,3,3a,4,6,6a-hexahydrofuro[3,4-c]furan-6-yl]-4-methoxy-1,3-benzodioxole (Verbindung 1): 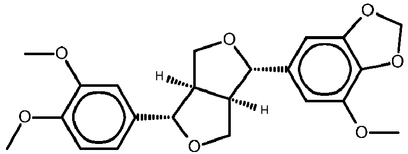
(+)-Diasesartemin (4-Methoxy-6-[(*1R,3aR,4S,6aR*)-tetrahydro-4-(3,4,5-trimethoxyphenyl)-1H, 3H-furo[3,4-c]furan-1-yl]-1,3-benzodioxol) (Verbindung 2): 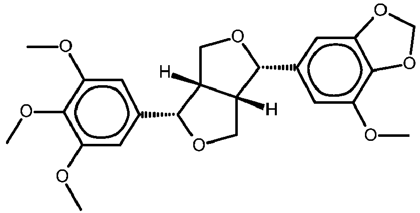
(+)-Sesartemin (6-[(*3R,3aS,6R,6aS*)-6-(3,4,5-Trimethoxyphenyl)-1,3,3a,4,6,6a-hexahydrofuro [3,4-c] furan-3-yl]-4-methoxy-1,3-benzodioxol) (Verbindung 3): 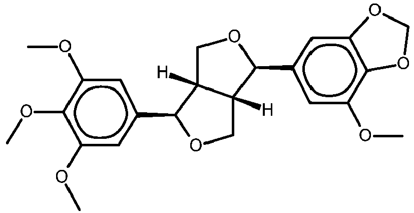
(+)-Episesartemin B (6-[(*3S,3aR,6R,6aR*)-6-(3,4,5-Trimethoxyphenyl)-1,3,3a,4,6,6a-hexahydro furo [3,4-c]furan-3-yl]-4-methoxy-1,3-benzodioxol) (Verbindung 4): 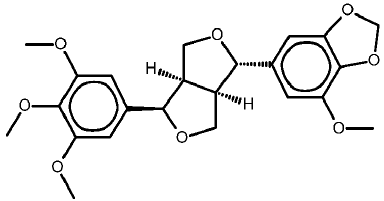
(+)-Episesartemin A (6-[(*3R,3aR,6S,6aR*)-6-(3,4,5-Trimethoxyphenyl)-1,3,3a,4,6,6a-hexahydro furo [3,4-c]furan-3-yl]-4-methoxy-1,3-benzodioxol) (Verbindung 5): 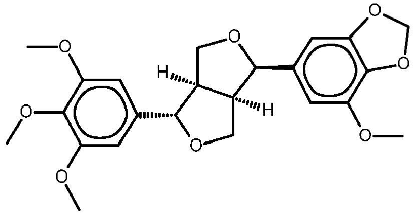
(+)-Diayangambin ((*3R,3aR,6R,6aR*)-3,6-Bis(3,4,5-trimethoxyphenyl)-1,3,3a,4,6,6a-hexahydro furo [3,4-c]furan) (Verbindung 6):

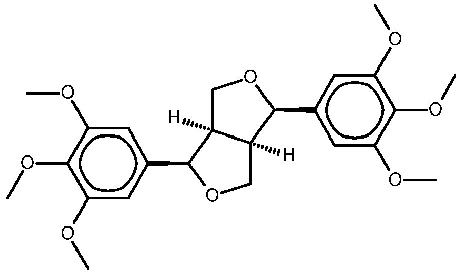

Die Tetrahydrolignane der Formel (I) können synthetisch oder biotechnologisch hergestellt oder aus geeignetem Pflanzenmaterial angereichert oder isoliert werden. Die erfindungsgemäßen Verbindungen 1 bis 6 sind an sich bekannt und kommen in verschiedenen Gewürzpflanzen vor; dabei ist aber die regelmäßig vorkommende Konzentration in der Regel sehr gering, so dass die bevorzugten erfindungsgemäßen Dosierungen nicht durch einfaches Zumengen des Gewürz oder des einfachen Gewürzextraktes erreicht werden können. Vielmehr müssen die Verbindungen aus diesen Extrakten entsprechend angereichert oder isoliert oder synthetisch gewonnen werden. Es wurden zwar schon die Einzelsubstanzen angereichert oder isoliert, allerdings in keiner Weise sensorisch auf Geruchs- oder Geschmackseffekte untersucht.

6-[(3*S*,3*aR*,6*S*,6a*R*)-3-(3,4-Dimethoxyphenyl)-1,3,3a,4,6,6a-hexahydrofuro[3,4-c]furan-6-yl]-4-methoxy-1,3-benzodioxol (Verbindung 1) wurde in *Macropiper excelsum* identifiziert (Dissertation Katja Obst, TU München, 2014). (+)-Diasesartemin (Verbindung 2), (+)-Sesartemin (Verbindung 3), (+)-Episesartemin B (Verbindung 4) und Episesartemin A (Verbindung 5) wurden in *Artemisia absinthium* (Greger, H., Hofer, O., 1980. New unsymmetrically substituted tetrahydrofurofuran lignans from Artemisia absinthium: Assignment of the relative stereochemistry by lanthanide induced chemical shifts. Tetrahedron 36, 3551-3558), Verbindungen 3 und 4 auch in *Achillea holosericea* (Ahmed, A. A., Mahmoud, A. A., Ali, E. T., Tzakou, O., Couladis, M., Mabry, T. J., Gati, T., Toth, G., 2002. Two highly oxygenated eudesmanes and 10 lignans from Achillea holosericea. Phytochemistry 59, 851-856) beschrieben. (+)-Episesartemin A (Verbindung 5) wurde in *P. fimbriulatum* gefunden (Solis, P. N., Olmedo, D., Nakamura, N., Calderon, A. I., Hattori, M., Gupta, M. P., 2005. A New Larvicidal Lignan from Piper fimbriulatum. Pharmaceutical Biology 43, 378-381). (+)-Diayangambin (Verbindung 6) wurde aus M. *excelsum* (Russell, G. B., Fenemore, P. G., 1973. New lignans from leaves of Macropiper excelsum. Phytochemistry 12, 1799-1803), *Piper fimbriulatum* (Solis et al., 2005) und *Piper aborescens* (Duh, C.-Y., Wu, Y.-C., Wang, S.-K., 1990. Cytotoxic pyridone alkaloids from Piper aborescens. Phytochemistry 29, 2689-2691) isoliert.

Die Pflanzenextrakte enthalten eine oder mehrere der bevorzugt genannten Verbindungen und sind ebenfalls im Sinne der Erfindung einsetzbar, beispielsweise Extrakte aus *Macropiper excelsum, Artemisia absinthium, Achillea holosericea,* Pfefferextrakte *(Piper ssp.,* beispielsweise *Piper fimbriulatum* oder *Piper aborescens*). Die pflanzlichen Extrakte enthalten dabei eines oder mehrere der Tetrahydrofuranlignane der Formel I, insbesondere der Verbindungen 1 bis 6. Die Tetrahydrofuranlignane der Formel I können aus den entsprechenden frischen oder getrockneten Pflanzen oder Pflanzenteilen der oben genannten gewonnen werden. Üblicherweise werden die Pflanzenteile (z.B. frische oder getrocknete Wurzeln, Früchte, Samen, Rinde, Holz, Stängel, Blätter oder Blüten[teile]), vorzugsweise in zerkleinerter Form, mit einem für Nahrungs- und Genussmittel geeigneten Lösungsmittel bei Temperaturen von 0°C bis zum Siedepunkt des jeweiligen Lösungsmittels oder Lösungsmittelgemisches extrahiert, anschließend filtriert und das Filtrat ganz oder teilweise eingeengt, vorzugsweise durch Destillation, Gefrier- oder Sprühtrocknung. Der so erhaltene Rohextrakt kann dann noch weiter aufgearbeitet werden, beispielsweise enzymatisch behandelt werden, mit Säure (z.B. unter Druck), mit sauren Ionentauschern oder mit Wasserdampf behandelt werden, meist bei Drücken von 0,01 mbar bis 100 bar, vorzugsweise bei 1 mbar bis 20 bar, und/oder in einem für Nahrungs- und Genussmittel geeigneten Lösungsmittel aufgenommen werden. Bevorzugt sind Extrakte, in denen die Tetrahydrofuranlignane der Formel I den Hauptanteil bezogen auf die Gesamtmenge aller Verbindungen darstellen, beispielsweise 1 bis 90 %, bevorzugt 5 Gew.-%, besonders bevor-zugt 10 Gew.-%, besonders bevorzugt 50 Gew.-%, ganz besonders bevorzugt 80 Gew.-% bezogen auf die Gesamtmenge des Extrakts.

Demgemäß werden Tetrahydrofuranlignane der Formel (I) vorzugsweise in Form ihrer Extrakte eingesetzt. Daher ist in einer bevorzugten Ausführungsform die zur Anwendung eingesetzten Tetrahydrofuranlignane der Formel (I) ein Extrakt.

Vorzugsweise ist mindestens ein Tetrahydrofuranligan der Formel (I) zur Anwendung geeignet. Es können aber auch Mischungen aus zwei, drei, vier oder mehrere Tetrahydrolignane der Formel (I) verwendet werden.

### Aromazusammensetzung

Es wurde ferner überraschenderweise gefunden, dass Tetrahydrofuranlignane der allgemeinen Formel (I) oder ein Extrakt davon, nicht nur eine kühlende bzw. trigeminale Wirkung haben, sondern auch in einer Aromazusammensetzung, welche in Zubereitungen eingearbeitet wird, die als negativ empfundene Geschmacks- oder Aromawirkung von schlecht schmeckenden Stoffen, insbesondere bitter oder adstringierend schmeckenden Stoffen, verändern, mindern oder unterdrücken können. Ebenfalls zeigte sich überraschenderweise, dass Tetrahydrofuranlignane der allgemeinen Formel (I) oder ein Extrakt davon, in einer Aromazusammensetzung (welche keine schlecht schmeckenden Geschmacksnoten aufweisen), welche dann in einer Zubereitung eingearbeitet wurde, die als negativ empfundene Geschmackseindrücke von schlecht schmeckenden Stoffen, insbesondere bitter oder adstringierend schmeckenden Stoffen der Endzubereitung, verändern, mindern oder unterdrücken können.
Demgemäß ist ein weiterer Gegenstand der vorliegenden Erfindung eine Aromazusammensetzung, umfassend mindestens ein Tetrahydrofuranlignan der allgemeinen Formel (I).

Eine solche erfindungsgemäße Aromazusammensetzung umfasst vorzugsweise mindestens eine Verbindung der Formel (I), vorzugsweise können auch mehrere Verbindungen der Formel (I) eingesetzt werden. In einer bevorzugten Ausführungsform weist eine erfindungsgemäße Aromazusammensetzung mindestens eine, zwei oder mehrere Verbindungen der Formel (I) auf, wobei ein solches Gemisch, wie bereits oben beschrieben, unterschiedliche Stereoisomere (Enantiomere und Diastereomere) einer Verbindung umfassen kann. Demgemäß ist mit einer Verbindung der Formel (I) auch die einzelne bestimmt Verbindung der Formel (I) gemeint, mit einer bestimmten Stereoisomerenstruktur.

Ein weiterer Gegenstand der vorliegenden Erfindung ist eine Aromazusammensetzung, umfassend mindestens ein Tetrahydrofuranlignan der allgemeinen Formel (I) oder ein Extrakt davon: 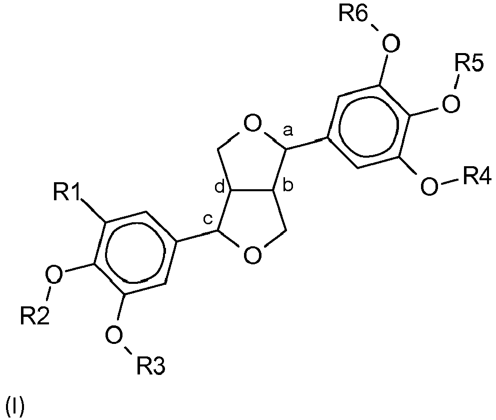
wobei R 1, R2, R3, R4, R5 und R6 unabhängig voneinander ausgewählt sind aus der Gruppe bestehend aus: Wasserstoff, C1-C3-Alkyl,
   und/oder
R2 und R3 jeweils zusammen und / oder
R5 und R6 jeweils zusammen
einen carbocyclischen Ring ausbilden können, ausgewählt aus der Gruppe bestehend aus: -CH2-, -CH2-CH2-, -CH2-CH2-CH2-, -CH2-CH2-CH2-CH2-,
   wobei
die Stereozentren a, b, c und d jeweils unabhängig voneinander eine(R)- oder (S)-Konfiguration aufweisen.

In einer bevorzugten Ausführungsform der Aromazusammensetzung, sind hierbei Verbindungen der Formel (I) bevorzugt, wobei
R1 Wasserstoff oder C1-C3-Alkyl ist, und
R2, R3, R4, R5, und R6 unabhängig voneinander ein C1-C3Alkyl darstellen,
   und/oder
R2 und R3 jeweils zusammen und/oder
R5 und R6 jeweils zusammen
einen carbocyclischen Ring ausbilden können, ausgewählt aus der Gruppe bestehend aus: - CH2-, -CH2-CH2-, -CH2-CH2-CH2-, -CH2-CH2-CH2-CH2-,
   wobei
die Stereozentren a, b, c und d jeweils unabhängig voneinander eine(R)- oder (S)-Konfiguration aufweisen.

In einer weiteren bevorzugten Ausführungsform der Aromazusammensetzung, sind hierbei Verbindungen der Formel (I) bevorzugt, wobei
R1 Wasserstoff oder C1-C3-Alkyl ist, und
R2, R3, R4, R5, und R6 unabhängig voneinander ein C1-C3Alkyl darstellen,
   und/oder
R5 und R6 jeweils zusammen
einen carbocyclischen Ring ausbilden können, ausgewählt aus der Gruppe bestehend aus: - CH2-, -CH2-CH2-, -CH2-CH2-CH2-, -CH2-CH2-CH2-CH2-,
   wobei
die Stereozentren a, b, c und d jeweils unabhängig voneinander eine(R)- oder (S)-Konfiguration aufweisen.

In einer bevorzugten Ausführungsform der Aromazusammensetzung, wird der carbocyclischer Ring der Tetrahydrofuranlignane der Formel (I) vorzugsweise durch R2 und R3 zusammen und/oder R5 undR6 zusammen ausgebildet, welche jeweils unabhängig voneinander ein Dioxolanring darstellen kann und wobei die C1-C3-Alkygruppe eine Methylgruppe ist.

In einer bevorzugten Ausführungsform der Aromazusammensetzung, sind die Verbindungen der Formel (I) vorzugsweise ausgewählt aus der Gruppe bestehend aus:
6-[((*3S,3aR,6S,6aR*)-3-(3,4-Dimethoxyphenyl)-1,3,3a,4,6,6a-hexahydrofuro[3,4-c]furan-6-yl]-4-methoxy-1,3-benzodioxole (Verbindung 1)
(+)-Diasesartemin (4-Methoxy-6-[(*1R,3aR,4S,6aR*)-tetrahydro-4-(3,4,5-trimethoxyphenyl)-1H,3H-furo[3,4-c]furan-1-yl]-1,3-benzodioxol)(Verbindung 2)
(+)-Sesartemin (6-[(*3R,3aS,6R,6aS*)-6-(3,4,5-Trimethoxyphenyl)-1,3,3a,4,6,6a-hexahydrofuro[3,4-c]furan-3-yl]-4-methoxy-1,3-benzodioxol)(Verbindung 3)
(+)-Episesartemin B (6-[(*3S,3aR,6R,6aR*)-6-(3,4,5-Trimethoxyphenyl)-1,3,3a,4,6,6a-hexahydrofuro[3,4-c]furan-3-yl]-4-methoxy-1,3-benzodioxol)(Verbindung 4)
(+)-Episesartemin A (6-[(*3R,3aR,6S,6aR*)-6-(3,4,5-Trimethoxyphenyl)-1,3,3a,4,6,6a-hexahydrofuro[3,4-c]furan-3-yl]-4-methoxy-1,3-benzodioxol)(Verbindung 5)
(+)-Diayangambin ((*3R*,*3aR*,*6R*,*6aR*)-3,6-Bis(3,4,5-trimethoxyphenyl)-1,3,3a,4,6,6a-hexahydrofuro[3,4-c]furan)(Verbindung 6).

Ein weiterer Gegenstand der vorliegenden Erfindung betrifft Aromazusammensetzungen, umfassend
(a) mindestens ein Tetrahydrofuranlignan der allgemeinen Formel (I) oder ein Extrakt davon, vorzugsweise eine Mischung aus mehreren Tetrahydrofuranlignane der allgemeinen Formel (I),
(b) mindestens eine weitere Verbindung mit physiologischer Kühlwirkung, wobei die weitere Verbindung (b)
   (i) einen geschmacklichen Effekt bewirkt oder
   (ii) keinen geschmacklichen Effekt bewirkt,
(c) mindestens einen weiteren Aromastoff ohne physiologische Kühlwirkung und/oder
(d) mindestens einen trigeminalen oder mundwässernd wirksamen Stoff ohne physiologische Kühlwirkung.

Geeignete Verbindungen (b) mit physiologischer Kühlwirkung sind vorzugsweise ausgewählt aus der Gruppe bestehend aus: Menthol und Mentholderivate (z.B. L-Menthol, D-Menthol, racemisches Menthol, Isomenthol, Neoisomenthol, Neomenthol) Menthylether (z.B. (I-Menthoxy)-1,2-propandiol, (I-Menthoxy)-2-methyl-1,2-propandiol, I-Menthyl-methylether), Menthylester (z.B. Menthylformiat, Menthylacetat, Menthylisobutyrat, Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat oder deren Gemischen), die Halbester von Mentholen mit einer Dicarbonsäure oder deren Derivate (z.B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), Avenanthramide, Menthancarbonsäureamide (z.B. Menthancarbonsäure-N-ethylamid [WS3], N^{α}-(Menthancarbonyl)glycinethylester [WS5], Menthancarbonsäure-N-(4-cyanophenyl)amid, Menthancarbonsäure-N-(4-methoxyphenyl)amid, Menthancarbonsäure-N-(alkoxyalkyl)amide), Menthon und Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid [WS23]), Isopulegol oder seine Ester (I-(-)-Isopulegol, I-(-)-Isopulegolacetat), Menthanderivate (z.B. p-Menthan-3,8-diol), Cubebol oder synthetische oder natürliche Mischungen, enthaltend Cubebol, Pyrrolidonderivate von Cycloalkyldionderivaten (z.B. 3-Methyl-2(1-pyrrolidinyl)-2-cyclopenten-1-on) wie in US 2004 052,828 beschrieben oder Tetrahydropyrimidin-2-one (z.B. Icilin oder verwandte Verbindungen), Zimtsäureamide, Spirokomponenten, Phenoxyesiggsäureamide.

Geeignete Verbindungen (b) mit physiologischer Kühlwirkung, jedoch ohne gleichzeitig einen geschmacklichen Effekt bewirkt sind vorzugsweise ausgewählt aus der Gruppe bestehend aus: Menthylether (z.B. (I-Menthoxy)-1,2-propandiol, (I-Menthoxy)-2-methyl-1,2-propandiol), polarere Menthylester (z.B. Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat), die Halbester von Mentholen mit einer Dicarbonsäure oder deren Derivate (z.B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), Avenanthramide wie in WO 2006 117,602 beschrieben, Menthancarbonsäureamide (z.B. Menthancarbonsäure-N-ethylamid [WS3], N^{α}-(Menthancarbonyl)glycinethylester [WS5], Menthancarbonsäure-N-(4-cyanophenyl)amid, Menthancarbonsäure-N-(4-methoxyphenyl)amid, Menthancarbonsäure-N-(alkoxyalkyl)amide), Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid), Pyrrolidonderivate von Cycloalkyldionderivaten (z.B. 3-Methyl-2(1-pyrrolidinyl)-2-cyclopenten-1-on) oder Tetrahydropyrimidin-2-one (z.B. Icilin oder verwandte Verbindungen), Zimtsäureamide, Spirokomponenten, Phenoxyesiggsäureamide.

Geeignete Verbindungen (b) mit physiologischer Kühlwirkung, die gleichzeitig einen geschmacklichen Effekt bewirkt sind vorzugsweise ausgewählt aus der Gruppe bestehend aus: polare Menthylester (z.B. Menthyllactate, L-Menthyl-L-lactat, L-Menthyl-D-lactat), die Halbester von Mentholen mit einer Dicarbonsäure oder deren Derivate (z.B. Mono-Menthylsuccinat, Mono-Menthylglutarat), Avenanthramide.

Bevorzugte Aromastoffe (c) ohne physiologische Kühlwirkung sind die Aromastoffe, die zu ihrem eigentlichen geruchlichen Aromawert auch einen Geschmackseindruck, einen geschmacksmodulierenden Effekt oder einen trigeminalen, aber nicht-kühlenden oder auch einen mundwässernden Reiz vermitteln. Bevorzugte Geschmackseindrücke sind süß, umami, bitter, salzig und sauer; bevorzugte geschmacksmodulierende Effekte sind bittermaskierende, adstringenz-maskierende, umami-verstärkende, süß-verstärkende, salzverstärkende und sauer-maskierende Effekte; bevorzugte trigeminale Reize sind Schärfe, Wärme, Kribbeln und Stechen. Besonders bevorzugte Aromastoffe (c) sind z.B. Pellitorine, vorzugsweise 2E,4E-Dodecadiensäure-N-isobutylamid (Kalecide) (2), 2E,4E-Tetradecadiensäure-N-isobutylamid (3), Spilanthol und Sanshoole.

Besonders bevorzugt ist eine Aromazusammensetzung, in der die weitere Verbindung (b) mit physiologischer Kühlwirkung, keinen geschmacklichen Effekt und keine Aromawirkung bewirkt, so dass lediglich eine Kühlwirkung (im Wesentlichen) ohne weiteren sensorischen Effekt vermittelt wird. Hierdurch wird vorzugsweise vermieden, dass ein Aromaprofil der erfindungsgemäßen Aromazusammensetzung beispielsweise in Richtung "Minze" (Pfefferminze) gebunden wird.

Ganz besonders bevorzugt ist eine Aromazusammensetzung, in der der weitere Aromastoff (c) und / oder der/die mindestens einen trigeminalen oder mundwässernden wirksamen Stoff (d) ohne physiologische Kühlwirkung unabhängig voneinander oder gemeinsam zusätzlich einen geschmacksmodulierenden Effekt und/oder einen trigeminalen und/oder einen mundwässernden Reiz vermitteln, wobei der trigeminale Reiz bevorzugt keine physiologische Kühlwirkung ist. Insbesondere weisen erfindungsgemäße Aromazusammensetzungen, die sowohl Aromastoffe (c) als auch Verbindungen (d) enthalten, eine angenehme Kühlwirkung mit ausgeglichenem sensorischen Profil auf, bei gleichzeitigem hohem Impact, d.h. hohem geschmacklichen Ersteindruck.

### Zubereitungen

Ein weiterer Gegenstand der vorliegenden Erfindung sind Zubereitungen, umfassend mindestens
(i) ein Tetrahydrofuranlignan der allgemeinen Formel (I) oder
(ii) ein Extrakt davon oder
(iii) eine erfindungsgemäße Aromazusammensetzung, umfassend mindestens ein Tetrahydrofuranlignan der allgemeinen Formel (I), wie bereits oben beschrieben,
wobei die Zubereitung ein Nahrungs-, Lebens- oder Genussmittel, pharmazeutisches oder kosmetisches Produkt, eine Halbfertigware oder ein Mundhygenie Produkt ist.

Vorzugsweise betrifft eine solche Zubereitung die Ernährung, der Mundhygiene oder dem Genuss dienende oder pharmazeutische oder kosmetische Zubereitung, die eine zum Erreichen einer physiologischen Kühlwirkung auf der Haut und/oder Schleimhaut ausreichende Menge aufweist an
(a) Tetrahydrofuranlignane der allgemeinen Formel (I) oder ein Extrakt davon, oder eine erfindungsgemäße Aromazusammensetzung, umfassend mindestens ein Tetrahydrofuranlignan der allgemeinen Formel (I), wie bereits oben beschrieben,
   und
(b) mindestens einen weiteren geeigneten Bestandteil ausgewählt aus den üblichen Grund-, Hilfs- und Zusatzstoffen.

Die erfindungsgemäßen Zubereitungen stellen gebrauchs- (der Mundhygiene oder pharmazeutische oder kosmetische dienende) oder verzehrfertige (der Ernährung oder dem Genuss dienenden) Zubereitungen dar. Dabei handelt es sich um Produkte, die dazu bestimmt sind, auf die menschliche Haut oder Schleimhaut oder in die menschliche Mundhöhle eingebracht zu werden, dort eine bestimmte Zeit zu verbleiben (leave-on kosmetische Zubereitungen) und anschließend entweder abgewaschen (wash off kosmetische Zubereitungen), verzehrt (z.B. verzehrfertige Lebensmittel, siehe auch weiter unten) oder wieder aus der Mundhöhle entfernt zu werden (z.B. Kaugummis). Zu diesen Produkten gehören dabei sämtliche Stoffe oder Erzeugnisse, die dazu bestimmt sind, in verarbeitetem, teilweise verarbeitetem oder unverarbeitetem Zustand vom Menschen aufgenommen zu werden. Hierzu zählen auch Stoffe, die kosmetischen Mitteln oder Lebensmitteln bei ihrer Herstellung, Verarbeitung oder Bearbeitung zugesetzt werden und dazu vorgesehen sind, auf die menschliche Haut oder in die menschliche Mundhöhle eingebracht zu werden.

Insbesondere soll die eingesetzte Menge von Tetrahydrofuranlignane der allgemeinen Formel (I) oder ein Extrakt davon, zum Erreichen einer physiologischen Kühlwirkung auf der Schleimhaut im Mund-, Nasen- und/oder Rachenraum ausreichen.

Bevorzugte erfindungsgemäße Zubereitungen umfassen
(a) 0,0001 Gew.-% bis 20 Gew.-%, bevorzugt 0,0001 bis 10 Gew.-%, besonders bevorzugt 0,001 Gew.-% bis 0,5 Gew.-% Tetrahydrofuranlignane der allgemeinen Formel (I) oder ein Extrakt davon, bezogen auf das Gesamtgewicht der Zubereitung und
(b) mindestens einen weiteren geeigneten Bestandteil ausgewählt aus üblichen Grund-, Hilfs- und Zusatzstoffen.

Wird eine Aromamischung eingesetzt, so ist in einer erfindungsgemäße Zubereitung die Summer aller Tetrahydrofuranlignane der Formel (I) im Bereich von 0,0001 Gew.-% bis 20 Gew.-%, bevorzugt 0,0001 bis 10 Gew.-%, besonders bevorzugt 0,001 Gew.-% bis 0,5 Gew.-%, bezogen auf das Gesamtgewicht der Zubereitung.

Soweit die vorliegende Erfindung eine Verwendung in entsprechenden Zubereitungen betrifft, sind dabei vorzugsweise solche Zubereitungen auszuwählen, die adstringierend schmecken und ein oder mehrere adstringieren schmeckende Stoffe enthält. Solche bevorzugten oral konsumierbare Produkte können z.B. sein: auf Schwarz- oder Grüntee basierende Produkte, auf Catechin- und/oder Tannin-reichen, adstringierendend schmeckenden Pflanzenteilen (z.B. Früchte bestimmter Rosacea-Arten, bestimmte Apfelarten, Weintrauben, Wein, Traubenkernextrakten, Rhabarber, Amla, Kakao, Mate) beruhende Produkte, auf Soja basierende Produkte, fettreduzierte Produkte, z.B. fettreduzierte Milchprodukte, insbesondere fettreduzierte Joghurts darstellen.

Auf Schwarz- oder Grüntee basierende, Catechin-haltige Zubereitungen im Sinne der Erfindung sind insbesondere Produkte enthaltend Schwarz- oder Grüntee-Extrakte, z.B. Eistees oder funktionelle Teegetränke, oder Produkte enthaltend oder angereichert mit Fraktionen oder Einzelstoffe aus Tee oder Teeextrakten, z.B. Catechine, insbesondere Epigallocatechingallat.

Auf Soja basierende Produkte im Sinne der Erfindung sind insbesondere Produkte aus Sojaprotein oder anderen Sojabohnen-Fraktionen, wie z. B. Sojamilch und daraus gefertigte Produkte, isolierte oder enzymatisch behandeltes Sojaprotein enthaltende Getränke, Sojamehl enthaltende Getränke, Sojalezithin-haltige Zubereitungen, fermentierte Produkte wie Tofu oder Tempe oder daraus gefertigte Produkte und Mischungen mit Fruchtzubereitungen und falkutativ Aromen.

Fettreduziert Milchprodukte, insbesondere fettreduzierte Joghurts sind im Sinne fettreduzierter, bzw. fettarmer Produkte zu verstehen und somit den wie weiter oben gemäß der erwähnten EG-Verordnung definierten Produkten zuzuordnen.

Folglich sind solche Zubereitungen besonders bevorzugt, die einen oder mehrere unangenehm schmeckende Stoffe (wie oben definiert) umfassen, wobei der oder die unangenehm schmeckenden Stoffe einen, zwei, drei, vier, fünf oder sämtliche der Geschmackseindrücke adstringierend, trocken, staubig, mehlig, kalkig oder metallisch aufweisen.

Wie sich in unseren Untersuchungen herausstellte, sind erfindungsgemäße Zubereitungen besonders bevorzugt, die dadurch gekennzeichnet sind, dass der oder die unangenehm schmeckenden Stoffe ausgewählt sind aus der Gruppe bestehend aus: Xanthinalkaloide, Xanthine (Coffein, Theobromin, Theophyllin und Methylxanthine), Alkaloide (Chinin, Brucin, Strychnin, Nicotin), phenolische Glycoside (z.B. Salicin, Arbutin), Flavonoidglycoside (z.B. Neohespereidin, Hesperidin, Naringin, Quercitrin, Rutin, Hyperosid, Quercetin-3-O-glucosid, Myricetin-3-O-glycoside), Chalcone oder Chalconglycoside (z.B. Phloridzin, Phloridzinxyloside), hydrolisierbare Tannine (Gallus- oder Elagsäureester von Kohlenhydraten, z.B. Pentagalloylglucose, Tanninsäuren), nichthydrolisierbare Tannine (ggfs. galloylierte Catechine, Gallocatechine, Epigaloocatechine oder Epicatechine und deren Oligomeren, z.B. Proanthyocyanidine oder Procyanidine, Thearubigenin), Flavone (z.B. Quercetin, Taxifolin, Myricetin), Phenole wie z.B. Salicin, Polyphenole (z.B. γ-Oryzanol, Kaffeesäure oder deren Ester (z. B. Chlorogensäure und Isomere)), terpenoide Bitter- und Gerbstoffe (z.B. Limonoide wie Limonin oder Nomilin aus Zitrusfrüchten, Lupolone und Humolone aus Hopfen, Iridoide, Secoiridoide), Absinthin aus Wermut, Amarogentin aus Enzian, metallische Salze (insbesondere Kalium-, Magnesium- und Calciumsalze, Kaliumchlorid, Kaliumgluconat, Kaliumcarbonat, Kaliumsulfat, Kaliumlactat, Kaliumglutamat, Kaliumsuccinat, Kaliummalat, Natriumsulfat, Magnesiumsulfat, Aluminiumsalze, Zinksalze, Zinnsalze, Eisen-(II)-salze, Eisen-(III)-salze, Chrom-(II)-picolinat), pharmazeutische Wirkstoffe (z.B. Fluorchinolon-Antibiotika, Paracetamol, Aspirin, β-Lac¬tam-Antibiotika, Ambroxol, Propylthiouracil [PROP], Guaifenesin), Vitamine (beispielsweise Vitamin H, Vitamine aus der B-Reihe wie Vitamin B1, B2, B6, B12, Niacin, Panthotensäure), Denatoniumbenzoat, Sucraloseoctaacetat, Eisensalze, Aluminiumsalze, Zinksalze, Harnstoff, ungesättigte Fettsäuren, insbesondere ungesättigte Fettsäuren in Emulsionen, bitter/adstringierend schmeckende Aminosäuren (z.B. Leucin, Isoleucin, Valin, Tryptophan, Prolin, Histidin, Tyrosin, Lysin oder Phenylalanin) und bitter oder adstringierend schmeckende Peptide oder Proteine (insbesondere Peptide mit einer Aminosäure aus der Gruppe Leucin, Isoleucin, Valin, Tryptophan, Prolin oder Phenylalanin am N- oder C-Terminus), Saponine, insbes. Sojasaponine, Isoflavonoide (insbes. Genistein, Daidzein, Genistin, Daidzin, deren Glycoside und acylierten Glycoside).

Stoffe, die insbesondere einen adstringierenden, trockenen, staubigen, mehligen, kalkigen und/oder metallischen Nachgeschmack haben, können Aroma- oder Geschmackstoffe mit einem nicht unangenehmen Primärgeschmack (beispielsweise süß, salzig, würzig, sauer) und/oder -geruch sein, und z.B. zur Gruppe der Süßstoffe, Zuckeraustauschstoffe oder der Aromastoffe gehören. Beispielsweise seien genannt: Kaliumsalze (insbesondere Kaliumchlorid, Kaliumgluconat, Kaliumcarbonat, Kaliumsulfat, Kaliumlactat, Kaliumglutamat, Kaliumsuccinat, Kaliummalat), Aspartam, Acesulfam K, Neotam, Superaspartam, Saccharin, Sucralose, Tagatose, Monellin, Stevioside, Rebaudioside, Hernandulcin, Thaumatin, Miraculin, Glycyrrhizin, Glycyrrhetinsäure oder deren Derivate, Cyclamat oder die pharmazeutisch akzeptablen Salze der vorgenannten Verbindungen.

Die oben genannte Auswahl der für die erfindungsgemäßen Zubereitungen besonders geeigneten unangenehm schmeckenden Stoffe, bzw. der Stoffe mit entsprechendem Nachgeschmack, gilt natürlich gleichermaßen für die weiter oben definierten erfindungsgemäßen Verwendungen.

### Zubereitungen zur Mundhygiene

Der Mundhygiene dienende Zubereitungen sind insbesondere Zahnpflegemittel wie Zahnpasten, Zahngele, Zahnpulver, Mundwässer, Kaugummis und andere Mundpflegemittel. Zahnpflegemittel (als Basis für der Mundpflege dienende Zubereitungen), welche die Tetrahydrofuranlignane der Formel (I) enthalten, umfassen im allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite^{®}, Süßstoffe, wie z.B. Saccharin, Natrium-Cyclamat, Sucralose, Acesulfam-K oder Zuckeralkohole, Geschmackskorrigenzien für unangenehme Geschmackseindrücke wie z.B Hydroxyflavanone nach US 2002/0188019, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin und Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Kaugummis (als weiteres Beispiel für der Mundpflege dienende Zubereitungen), die Tetrahydrofuranlignane der Formel (I) enthalten, umfassen im allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkohole, Geschmackskorrigenzien für unangenehme Geschmackseindrücke, andere Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Aromen und Stabilisatoren oder Geruchskorrigentien.

### Nahrungsmittel

Im Rahmen des vorliegenden Textes werden unter einem "Nahrungs- oder Lebensmittel" insbesondere Stoffe verstanden, die dazu bestimmt sind, in unverändertem, zubereitetem oder verarbeitetem Zustand vom Menschen geschluckt und dann verdaut zu werden; als Lebensmittel werden insoweit auch Umhüllungen, Überzüge oder sonstige Umschließungen verstanden, die dazu bestimmt sind, mitverschluckt zu werden, oder bei denen ein Verschlucken vorauszusehen ist. Auch bestimmte Produkte, die üblicherweise wieder aus der Mundhöhle entfernt werden (z.B. Kaugummis) sind im Rahmen des vorliegenden Textes als Lebensmittel zu verstehen, da bei ihnen nicht auszuschließen ist, dass sie zumindest teilweise geschluckt werden.

Unter einem verzehrfertigen Lebensmittel ist hierbei ein Lebensmittel zu verstehen, das hinsichtlich der für den Geschmack maßgeblichen Substanzen bereits vollständig zusammengesetzt ist. Unter den Begriff "verzehrfertiges Lebensmittel" fallen auch Getränke sowie feste oder halbfeste verzehrfertige Lebensmittel. Als Beispiele seien genannt Tiefkühlprodukte, die vor dem Verzehr aufgetaut und auf Verzehrtemperatur erwärmt werden müssen. Auch Produkte wie Joghurt oder Eiscreme aber auch Kaugummis oder Hartkaramellen zählen zu den verzehrfertigen Lebensmitteln.

### Halbfertigwaren

Der Begriff der Zubereitung umfasst im Sinne der Erfindung auch Halbfertigwaren zur Herstellung einer Ernährung, der Mundhygiene oder dem Genuss dienende oder pharmazeutische oder kosmetische Zubereitung. Unter einer Halbfertigware ist im Zusammenhang mit der vorliegenden Erfindung ein Produkt zu verstehen, welches aufgrund eines sehr hohen Gehaltes an Aroma- und Geschmacksstoffen für die Verwendung als verzehrfertiges Lebensmittel oder gebrauchsfertige kosmetische oder pharmazeutische Zubereitung ungeeignet ist. Erst durch Mischen mit mindestens einem weiteren Bestandteil (d.h. durch Verringern der Konzentration der betreffenden Aroma- und Geschmacksstoffe) und gegebenenfalls weitere Prozessschritte (z.B. Erwärmen, Gefrieren) wird die Halbfertigware in ein verzehrfertiges Lebensmittel oder eine gebrauchsfertige kosmetische oder pharmazeutische Zubereitung überführt. Als Beispiel für Halbfertigwaren seien hier Aromamischungen, Aromakonzentrate, Tütensuppen, Backaromen und Puddingpulver genannt.

Die Halbfertigwaren können die Tetrahydrofuranlignane der Formel (I) oder ein Extrakt davon, im Bereich von 50 bis 700.000 ppm, bevorzugt 100 bis 500.000 ppm, besonders bevorzugt 1000 bis 400.000 ppm umfassen, bezogen auf das Gesamtgewicht. Bei Halbfertigwaren, werden diese in der Regel in einer Konzentration von 0,01 bis 10 Gew.-%, bevorzugt 0,05 bis 5 Gew.-%, besonders bevorzugt 0,05 bis 1 Gew.-%, bezogen auf das verzehrsfertige Lebensmittel oder eine gebrauchsfertige kosmetische oder pharmazeutische Zubereitung (Endprodukt) eingesetzt.

### Pharmazeutische Zubereitungen

Ein weiterer Gegenstand der vorliegenden Erfindung sind pharmazeutische Zubereitungen, umfassend
(i) mindestens ein Tetrahydrofuranlignan der allgemeinen Formel (I) oder ein Extrakt davon,oder
(ii) eine erfindungsgemäße Aromazusammensetzung, umfassend mindestens ein Tetrahydrofuranlignan der allgemeinen Formel (I),
(iii) mindestens ein pharmazeutisches akzeptables Trägermaterial.

Bevorzugte erfindungsgemäße pharmazeutische Zubereitungen im Sinne der Erfindung sind orale Zubereitungen, die z.B. in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen und als verschreibungspflichtige, apothekenpflichtige oder sonstige Arzneimittel oder als Nahrungsergänzungsmittel verwendet werden.

### Kosmetische Zubereitungen

Ein weiterer Gegenstand der vorliegenden Erfindung sind kosmetische Zubereitungen, umfassend
(i) mindestens ein Tetrahydrofuranlignan der allgemeinen Formel (I) oder ein Extrakt davon, oder
(ii) eine erfindungsgemäße Aromazusammensetzung, umfassend mindestens ein Tetrahydrofuranlignan der allgemeinen Formel (I),
(iii) mindestens ein kosmetisches akzeptables Trägermaterial.

Erfindungsgemäße kosmetische Zubereitungen können z. B. in einer der folgenden Formen vorliegen: Seife, Syndet, flüssiges Wasch-, Dusch-, oder Badepräparat, Emulsion (als Lösung, Dispersion, Suspension, Creme, Lotion oder Milch je nach Herstellungsverfahren und Inhaltsstoffen vom Typ "Wasser-in-Öl" (W/O), "Öl-in-Wasser" (O/W) oder multiple Emulsion, PIT Emulsion, Emulsionsschaum, Mikroemulsion, Nanoemulsion, Pickering Emulsion), Salbe, Paste, Gel (inklusive Hydro-, Hydrodispersions-, Oleogel), Öl, Toner, Balsam, Serum, Puder, Eau de Toilette, Toilette, Eau de Cologne, Perfum, Wachs, als Stift, Roll-On, (Pump-)Spray, Aerosol (schäumend, nicht schäumend oder nach-schäumend), Fusspflegemittel (inklusive Keratolytika, Desodorant), Bartreinigungs- oder -pflegemittel, Insekten abwehrendes Mittel, Sonnenschutzmittel, Aftersun-Präparat, Rasiermittel (z.B. Rasierschäume, -seifen oder -gele) oder After-Shave (Balm, Lotion), Haarentfernungsmittel, Haarpflegemittel wie z.B. Shampoo (z.B. 2-in-1 Shampoo, Anti-Schuppen-Shampoo, Babyshampoo, Shampoo für trockene Kopfhaut, Shampookonzentrat), Conditioner, Haarkur, Haarwasser, Haarspülung, Frisiercreme, Pomade, Dauerwell- und Fixierungsmittel, Haarglättungsmittel (Entkräuselungsmittel, Relaxer), Haarfestiger, Styling-Hilfe (z.B. Gel oder Wachs), Blondiermittel, Haaraufheller, Haarconditioner, Haarmousse, Haartönung, Haarfärbemittel (z.B. temporäre, direktziehende, semipermanente, permanente Haarfärbemittel), Nagelpflegemittel wie z.B. Nagellack und Nagellackentferner, Deodorant und / oder Antitranspirant, Mundwasser, Munddusche, Make-Up, Make-Up-Entferner, Augenpflege, Lippenkosmetika, Lippenpflegemittel, dekorative Kosmetik (z.B. Puder, Lidschatten, Kajalstift, Lippenstift), Badeartikel (z.B. Kapsel), oder Maske.

### Herstellung der Zubereitungen

Die erfindungsgemäßen Zubereitungen oder Halbfertigwaren werden vorzugsweise hergestellt, indem die Tetrahydrofuranlignane der Formel (I) oder ein Extrakt davon in Ethanol und gegebenenfalls demineralisiertem und/oder gereinigtem Wasser gelöst bzw. gemischt werden. Anschließend werden die Lösungen durch einen Trocknungsprozess, vorzugsweise einen Sprühtrocknungs-, Vakuumgefriertrockungs-, Umkehrosmose-, Eindampf- oder anderen Konzentrationsprozess oder eine Kombination der genannten Prozesse, in eine (zumindest nahezu) feste Form überführt. Dabei kann die Trocknung unter Zuhilfenahme von Trägerstoffen (z.B. Stärke, Stärkederivate, Maltodextrin, Kieselgel, siehe oben) oder Hilfsstoffen (z.B. Pflanzengummen, Stabilisierungsmittel) erfolgen. Vorzugsweise erfolgt die Trocknung mittels Sprühtrocknung oder Vakuumgefriertrocknung.

Gemäß einer weiteren bevorzugten Ausführungsform werden zur Herstellung erfindungsgemäßer Zubereitungen oder Halbfertigwaren Tetrahydrofuranlignane der Formel (I) oder ein Extrakt davon sowie gegebenenfalls andere Bestandteile zunächst in Emulsionen, in Liposomen (z.B. ausgehend von Phosphatidylcholin) in Microsphären, in Nanosphären oder auch in Kapseln, Granulate oder Extrudate aus einer für Lebens- und Genussmittel geeigneten Matrix (z.B. aus Stärke, Stärkederivaten, Cellulose oder Cellulosederivaten wie Hydroxypropylcellulose, anderen Polysacchariden wie Alginat, natürlichen Fetten, natürlichen Wachsen wie Bienenwachs oder Carnaubawachs oder aus Proteinen wie Gelatine) eingearbeitet.

In einem weiteren bevorzugten Herstellungsverfahren werden Tetrahydrofuranlignane der Formel (I) oder ein Extrakt davon mit einem oder mehreren geeigneten Komplexbildnern, beilspielsweise mit Cyclodextrinen oder Cyclodextrinderivaten, bevorzugt alpha- oder beta Cyclodextrin, komplexiert und in dieser komplexierten Form eingesetzt.

Besonders bevorzugt sind erfindungsgemäße Zubereitungen oder Halbfertigwaren, bei denen die Matrix so gewählt ist, dass die Tetrahydrofuranlignane der Formel (I) oder ein Extrakt davon, insbesondere Mischungen der einzelnen Stereoisomere der Tetrahydrofuranlignane der Formel (I) und weitere Kühlwirkstoffe und/oder Aromen umfassen, verzögert von der Matrix freigegeben werden, so dass man eine langanhaltende Kühlwirkung erhält. Als Matrix können hier z.B. natürliche Fette, natürliche Wachse (z.B. Bienenwachs, Carnaubawachs) oder auch natürliche Ballaststoffe (Weizenfasern, Apfelfasern, Haferfasern, Orangenfasern) verwendet werden.

Tetrahydrofuranlignane der allgemeinen Formel (I) oder ein Extrakt davon sind im Rahmen der üblichen Formulierungen und Zubereitungsbedingungen im Bereich von pH 1 bis pH 12, insbesondere im Bereich von pH 4 bis pH 9, bezogen auf wasserhaltige Zubereitungen, hydrolysestabil, so dass sie in Zubereitungen lange haltbar sind, so dass auch die jeweilige Zubereitung selbst lange haltbar sind.

Hydrolysestabil bedeutet im Zusammenhang mit diesem Text, dass die untersuchten Verbindungen bei 40°C, bei pH 9 und/oder pH 3, bei Tageslicht und bei einem Wassergehalt von mindestens 5 Gew.-% nach 6 Monaten zu weniger als 10 Mol-% (bevorzugt nicht signifikant) hydrolisiert werden und darüber hinaus bevorzugt unter den üblichen Herstellungsbedingungen für die in diesem Text genannten Zubereitungen zu weniger als 10 %, (nochmals bevorzugt nicht signifikant) Hydrolysereaktionen zeigen.

Insbesondere für Nahrungs- und Genussmittel zur Extraktion geeignete Lösungsmittel sind Wasser, Ethanol, Methanol, 1,2-Propylenglycol, 1,3-Propylenglykol, Glycerin, Aceton, Dichlormethan, Essigsäureethylester, Diethylether, Hexan, Heptan, Triacetin, pflanzliche Öle oder Fette, superkritisches Kohlendioxid und deren Gemische.

Bevorzugte Hilfs- oder Trägerstoffe sind Maltodextrin, Stärke, natürliche oder künstliche Polysaccharide und/oder Pflanzengummen wie modifizierte Stärken oder Gummi Arabicum, für die Aromakompositionen zugelassene Lösungsmittel wie z.B. Ethanol, 1,2-Propylenglycol, 1,3-Propylenglykol, Wasser, Glycerin, Triacetin, Pflanzenöltriglyceride, färbende Mittel, z.B. zugelassene Lebensmittelfarbstoffe, färbende Pflanzenextrakte, Stabilisatoren, Konservierungsstoffe, Antioxidantien, Viskositäts-beeinflussende Stoffe.

Die genannten Zubereitungen der vorliegenden Erfindung können weitere übliche Inhaltstoffe und Additive enthalten, die geeignet sind für die jeweiligen Bereiche.

### A.Trägerstoffe

Eine Reihe von erfindungsgemäßen Zubereitungen oder Halbfertigwaren (wie oben beschrieben) ist besonders bevorzugt. So sind beispielsweise erfindungsgemäße Zubereitungen oder Halbfertigwaren gemäß einer bevorzugten Ausführungsform sprühgetrocknete Zubereitungen bzw. Halbfertigwaren, die als weitere zum Verzehr geeignete Bestandteile (unter anderem) feste Trägerstoffe umfassen.

Erfindungsgemäß bevorzugt sind erfindungsgemäße Zubereitungen oder Halbfertigwaren, die einen oder mehrere zum Verzehr geeignete feste Trägerstoffe umfassen, wobei, bezogen auf die Trockenmasse der Zubereitung bzw. Halbfertigware, das Gewichtsverhältnis der Gesamtmenge an Tetrahydrofuranlignane der Formel (I) zu der Gesamtmenge an zum Verzehr geeigneten festen Trägerstoffen vorzugsweise im Bereich von 1 : 10 bis 1 : 100000, bevorzugt im Bereich von 1 : 50 (vorzugsweise 1 : 100) bis 1 : 20000, besonders bevorzugt im Bereich von 1 : 100 bis 1 : 5000, liegt.

Weiter bevorzugt ist es, wenn dabei die Gesamtmenge von Tetrahydrofuranlignane der Formel (I) und zum Verzehr geeigneten festen Trägerstoffen bezogen auf das Gesamtgewicht der Zubereitung bzw. Halbfertigware im Bereich von 70 bis 100 Gew.-%, bevorzugt im Bereich von 85 bis 100 Gew.-%, liegt.

Vorteilhafte Trägerstoffe sind Siliciumdioxid (Kieselsäure, Kieselgel), Kohlenhydrate und/oder Kohlenhydratpolymere (Polysaccharide), Cyclodextrine, Stärken, abgebaute Stärken (Stärkehydrolysate), chemisch oder physikalisch modifizierte Stärken, modifizierte Cellulosen, Gummi Arabicum, Ghatti-Gummi, Traganth, Karaya, Carrageenan, Guarkernmehl, Johannisbrotkernmehl, Alginate, Pektin, Inulin oder Xanthan Gum. Bevorzugte Stärkehydrolysate sind Maltodextrine und Dextrine.

Erfindungsgemäß besonders bevorzugte Trägerstoffe sind Siliciumdioxid, Gummi Arabicum und Maltodextrine, wobei hier wiederum Maltodextrine mit DE-Werten im Bereich 5 bis 20 besonders bevorzugt sind. Es ist dabei unerheblich, welche Pflanze ursprünglich die Stärke zur Herstellung der Stärkehydrolysate geliefert hat. Besonders geeignet und leicht verfügbar sind jedoch Mais-basierende Stärken sowie Stärken aus Tapioka, Reis, Weizen oder Kartoffeln. Die Trägerstoffe können dabei auch als Fließhilfsmittel fungieren, wie beispielsweise Siliciumdioxid.

Die erfindungsgemäßen Zubereitungen oder Halbfertigwaren, die neben der oder den erfindungsgemäß zu verwendenden Tetrahydrofuranlignane der Formel (I) bzw. oben definierten Mischungen noch einen oder mehrere feste Trägerstoffe umfassen, können beispielsweise durch mechanische Mischvorgänge, wobei gleichzeitig auch eine Zerkleinerung der Partikel erfolgen kann, oder mittels Sprühtrocknung hergestellt werden. Besonders bevorzugt sind dementsprechend erfindungsgemäße Zusammensetzungen oder Halbfertigwaren, die feste Trägerstoffe umfassen und mittels Sprühtrocknung hergestellt sind.

Bevorzugte erfindungsgemäße, Trägerstoffe umfassende Zubereitungen und Halbfertigwaren, die mittels Sprühtrocknung hergestellt wurden, besitzen eine mittlere Partikelgröße im Bereich von 30 bis 300 µm und eine Restfeuchte von kleiner oder gleich 5 Gew.-%.

### B.Aromastoffe

Die erfindungsgemäßen oralen Zubereitungen können einen oder mehrere Aromastoffe enthalten. Typische Beispiele umfassen: Acetophenon, Allylcapronat, alpha-Ionon, beta-Ionon, Anisaldehyd, Anisylacetat, Anisylformiat, Benzaldehyd, Benzothiazol, Benzylacetat, Benzylalkohol, Benzylbenzoat, beta-Ionon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion^{®}), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cisdecadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

Typischerweise werden Aromastoffe als Aromakompositionen eingesetzt, welche mehrere Aromen enthalten. Geeignete Aromakompositionen enthalten vorzugsweise synthetische, natürliche oder naturidentische Aroma-, Riech- und Geschmacksstoffe, Reaktionsaromen, Raucharomen oder andere aromagebende Zubereitungen (z.B. Protein[teil]hydrolysate, Grillaromen, Pflanzenextrakte, Gewürze, Gewürzzubereitungen, Gemüse und/oder Gemüsezubereitungen) sowie gegebenenfalls geeignete Hilfs- und Trägerstoffe. In der Regel enthalten die Aromakompositionen mehr als einen der genannten Inhaltsstoffe.

Modulierende Aroma- und/oder Geschmackstoffe werden vorzugsweise ausgewählt aus der Gruppe bestehend aus Adenosin-5'-monophosphat, Cytidin-5'-monophosphat, Inosin-5'-monophosphat, und deren pharmazeutisch akzeptablen Salzen; Lactisolen; 2,4-Dihydroxybenzoesäure; 3-Hydroxybenzoesäure; Natriumsalzen, vorzugsweise Natriumchlorid, Natriumlactat, Natriumcitrat, Natriumacetat, Natriumgluconoat; Hydroxyflavanonen, wie zum Beispiel Eriodictyol, Homoeriodictyol, und deren Natriumsalzen; Hydroxybenzoesäureamiden, wie zum Beispiel 2,4-Dihydroxybenzoesäurevanillylamid, 2,4-Dihydroxybenzoesäure-N-(4-hydroxy-3-methoxybenzyl)amid, 2,4,6-Trihydroxybenzoesäure-*N-*(4-hydroxy-3-methoxybenzyl)amid, 2-Hydroxy-benzoesäure-*N*-4-(hydroxy-3-methoxy-benzyl)amid, 4-Hydroxybenzoesäure-*N*-(4-hydroxy-3-methoxybenzyl)-amid, 2,4-Dihydroxy-benzoesäure-*N*-(4-hydroxy-3-methoxybenzyl)amid-Mono-natriumsalz, 2,4-Dihydroxybenzoesäure-N-2-(4-hydroxy-3-methoxyphenyl)-ethyl-amid, 2,4-Dihydroxybenzoesäure-*N-*(4-hydroxy-3-ethoxybenzyl)amid, 2,4-Dihydroxybenzoesäure-*N*-(3,4-dihydroxybenzyl)amid und 2-Hydroxy-5-methoxy-N-[2-(4-hydroxy-3-methoxyphenyl)ethyl]amid; 4-Hydroxybenzoesäurevanillylamid; Hydroxydeoxybenzoinen, wie zum Beispiel 2-(4-Hydroxy-3-methoxyphenyl)-1-(2,4,6-trihydroxyphenyl)ethanon, 1-(2,4-Dihydroxyphenyl)-2-(4-hydroxy3-methoxyphenyl)ethanon, 1-(2-Hydroxy-4-methoxyphenyl)-2-(4-hydroxy-3-methoxyphenyl) ethanon); Hydroxyphenlaalkandionen, wie zum Beispiel Gingerdion-[2], Gingerdion-[3], Gingerdion-[4], Dehydrogingerdion-[2], Dehydrogingerdion-[3], Dehydrogingerdion-[4]); Diacetyltrimeren; γ-Aminobuttersäuren und Divanillinen; Bicyclo[4.1.0]heptan-7-carbonsäureamide; Cyclopropancarbonsäure(3-methyl-cyclohexyl)amide, aromatische *Neo-*Menthylamide; Geranylaminederivate der Oxalsäure sowie Neomenthylderivate.

Die Aromakompositionen können auch in Form von Reaktionsaromen (Maillard-Produkte) und/oder Extrakten bzw. ätherischen Ölen von Pflanzen oder Pflanzenteilen bzw. Fraktionen davon eingesetzt werden. Hierbei sind Extrakte bzw. ätherischen Ölen aus aromatischen Pflanzen der *Mentha* ssp. oder *Eucalyptus* ssp.bevorzugt.

### C. Fette und Öle

Gemäß einer weiteren bevorzugten Ausgestaltung der vorliegenden Erfindung können die oralen Zubereitungen Fette und Öle enthalten und dann als Wasser-in-Öl (W/O)-Emulsion vorliegen.

Neben der oder den erfindungsgemäß zu verwendenden Verbindungen der Formel (I) umfasst eine solche Emulsion Wasser, eine Ölphase, ein oder mehrere (verzehrbare) W/O-Emulgatoren, gegebenenfalls ein oder mehrere Antioxidantien und gegebenenfalls ein oder mehrere Stoffe zur Verstärkung einer antioxidativen Wirkung. Dabei gilt hinsichtlich bevorzugter Tetrahydrofuranlignane der Formel (I) und Mischungen davon das oben Gesagte entsprechend. Die Ölphase einer solchen erfindungsgemäßen W/O-Emulsion besteht aus oder umfasst vorzugsweise ein fettes Öl und/oder eine Aromakomposition (vorzugsweise eine wie oben beschriebene Aromakomposition).

Als fette Öle eignen sich beispielsweise Speiseöle, insbesondere Pflanzenöle. Geeignete fette Öle sind beispielsweise Borretschöl, Distelöl, Erdnussöl, Haselnussöl, Kokosöl, Kürbiskernöl, Leinöl, Maiskeimöl, Makadamianussöl, Mandelöl, Olivenöl, Palmkernöl, Pekannussöl, Pistazienkernöl, Rapsöl, Reiskeimöl, Sesamöl, Sojaöl, Sonnenblumenöl, Walnussöl oder Weizenkeimöl, oder daraus erhältliche Fraktionen. Es können auch flüssige Neutralester basierend auf mittelkettigen Fettsäuren und Glycerin verwendet werden, wie beispielsweise Miglyole (z.B. Miglyol 810, Miglyol 812). Bevorzugt sind Sonnenblumenöl, Palmkernöl und Rapsöl. Ferner bevorzugt werden fraktionierte Kokosöle verwendet, die hauptsächlich Fettsäurereste mit 6 bis 8 C-Atomen aufweisen. Diese zeichnen sich durch ihre Geschmacksneutralität sowie durch ihre gute Oxidationsstabilität aus.

Die erfindungsgemäßen W/O-Emulsionen eignen sich besonders zum Aufbringen auf Lebensmitteloberflächen, wobei die Lebensmittel vorzugsweise einen Wassergehalt von höchstens 10 Gew.-%, bevorzugt von höchstens 5 Gew.-% aufweisen. In einer bevorzugten Ausführungsform weist die erfindungsgemäße W/O-Emulsion bei Aufbringungstemperatur eine ausreichend niedrige Viskosität auf, so dass ein Aufbringen der W/O-Emulsion mittels Sprühen möglich ist. Bevorzugte Lebensmittel, auf deren Oberflächen eine erfindungsgemäße W/O-Emulsion aufgebracht werden kann sind beispielsweise Cracker, Chips (z.B. auf Basis von Kartoffeln, Mais, Getreide oder Brot), extrudierte Knabberartikel (Snacks, z.B. Flips) oder Laugengebäck (z.B. Salzstangen). Erfindungsgemäße W/O-Emulsionen werden regelmäßig in einer Menge von 0,5 bis 6 Gew.-% auf die Lebensmitteloberflächen aufgebracht, bezogen auf das Gesamtgewicht des Lebensmittels.

### D. W/O-Emulgatoren

Zur Stabilisierung der Fett- bzw. Öl/Wassergemische werden für Lebensmittel zugelassene W/O-Emulgatoren benötigt. Vorzugsweise wird der verzehrbare W/O-Emulgator gewählt aus der Gruppe bestehend aus Lecithin (E 322), Mono- und Diglyceride von Speisefettsäuren (E 471), Essigsäuremonoglyceride (E 472a), Milchsäuremonoglyceride (E 472b), Citronensäuremonoglyceride (E 472c), Weinsäuremonoglyceride (E 472d), Diacetylweinsäuremonoglyceride (E 472e), Sorbitanmonostearat (E 491).

### E. Antioxidantien

Die Zubereitungen können des Weiteren auch Antioxidantien enthalten. Geeignete Antioxidantien und Stoffe, welche die antioxidative Wirkung verstärken können sind insbesondere die natürlich vorkommenden Tocopherole und deren Derivate, Tocotrienole, Flavonoide, Ascorbinsäure und ihre Salze, alpha-Hydroxysäuren (z.B. Citronensäure, Milchsäure, Äpfelsäure, Weinsäure) und deren Na-, K- und Ca-Salze, aus Pflanzen isolierte Inhaltsstoffe, Extrakte bzw. Fraktionen davon, z.B. aus Tee, Grüntee, Algen, Traubenkernen, Weizenkeimen, Rosmarin, Oregano, Flavonoide, Quercetin, phenolische Benzylamine. Weiterhin sind als Antioxidantien Propylgallat, Octylgallat, Dodecylgallat, Butylhydroxyanisol (BHA), Butylhydroxytoluol (BHT), Lecithine, Mono- und Diglyceride von Speisefettsäuren verestert mit Citronensäure, Orthophosphate und Na-, K- und Ca-Salze der Monophosphorsäure und Ascorbylpalmitat geeignet.

### F. Weitere Hilfs- und Zusatzstoffe

Weitere Bestandteile einer erfindungsgemäßen, der Ernährung oder dem Genuss dienenden verzehrfertigen Zubereitung oder einer erfindungsgemäßen Halbfertigware können übliche Grund-, Hilfs- und Zusatzstoffe für Nahrungs- oder Genussmittel sein, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Kräuter, Nüsse, Gemüsesäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutathion), native oder prozessierte Proteine (z.B. Gelatine), Enzyme (z.B. Peptidasen), Nucleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (s. hierzu auch oben; z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carrageenan, Alginat), Konservierungsstoffe (z.B. Benzoesäure und deren Salze, Sorbinsäure und deren Salze), Antioxidantien (z.B. Tocopherol, Ascorbinsäure), Chelatoren (z.B. Citronensäure), organische oder anorganische Säuerungsmittel (z.B. Essigsäure, Phosphorsäure), zusätzliche Bitterstoffe (z.B. Chinin, Coffein, Limonin, Amarogentin, Humolone, Lupolone, Catechine, Tannine), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigenzien.

Bevorzugt enthalten erfindungsgemäße Zubereitungen oder Halbfertigwaren zudem erfindungsgemäße Aromazusammensetzungen (wie oben beschrieben), um den Geschmack und/oder den Geruch abzurunden und zu verfeinern.

### GEWERBLICHE ANWENDBARKEIT

Ein wichtiger Aspekt der vorliegenden Erfindung betrifft die Verwendung von Tetrahydrofuranlignane der allgemeinen Formel (I) oder ein Extrakt davon als Kühlwirkstoff. Insbesondere ist eine bevorzugte Ausführung dabei die Verwendung von Tetrahydrofuranlignane der allgemeinen Formel (I) oder ein Extrakt davon zur physiologischen Kühlung der Haut oder Schleimhaut, hierbei ist insbesondere bei der Schleimhaut die des Mund-, Nasen- und Rachenraumes gemeint.

Eine bevorzugte Ausführungsform der vorliegenden Erfindung betrifft hierbei die Verwendung
(i) von mindestens einem Tetrahydrofuranlignan oder einem Gemisch aus mehreren Tetrahydrofuranlignane der Formel (I) oder ein Extrakt davon, oder
(ii) einer erfindungsgemäßen Aromazusammensetzung, umfassend midestens ein Tetrahydrofuranlignan der Formel (I) oder
(iii) einer erfindungsgemäßen Zubereitung, welche vorzugsweise ein Nahrungs-, Lebens- oder Genussmittel, pharmazeutisches oder kosmetisches Produkt, eine Halbfertigware oder ein Mundhygenie Produkt ist,
zum Erzeugen einer Kühlwirkung auf der Haut oder Schleimhaut, zu
(a) anderen als therapeutischen Zwecken oder
(b) zum Herstellen eines Arzneimittels.

Ein weiterer Aspekt der Erfindung betrifft die Verwendung der Tetrahydrofuranlignane der Formel (I) oder ein Extrakt davon, welche einen oder mehrere Aromastoffe und/oder einen oder mehrere weitere Kühlwirkstoffe (Kühlwirkstoffe, bei denen es sich nicht um Tetrahydrofuranlignane der Formel (I) handelt) umfasst, als Halbfertigwaren (sogenannte Aromazusammensetzungen) zur Aromatisierung von unter Verwendung der Halbfertigwaren gefertigten Fertigwaren. Insbesondere werden hierbei mindestens ein, zwei, drei oder vier verschiedene (Stereoisomere) Verbindungen der Formel (I) bevorzugt eingesetzt.

Demgemäß betrifft die vorliegende Erfindung auch Tetrahydrofuranlignane der allgemeinen Formel (I) oder ein Extrakt davon als Medikament, das bevorzugt als Kühlwirkstoff eingesetzt wird: 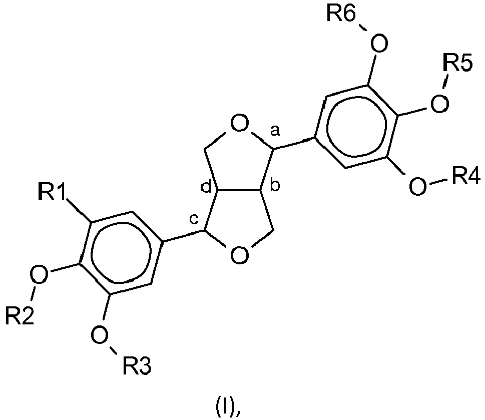
wobei R 1, R2, R3, R4, R5 und R6 unabhängig voneinander ausgewählt sind aus der Gruppe bestehend aus: Wasserstoff, C1-C3-Alkyl,
   und/oder
R2 und R3 jeweils zusammen und / oder
R5 und R6 jeweils zusammen
einen carbocyclischen Ring ausbilden können, ausgewählt aus der Gruppe bestehend aus: -CH2-, -CH2-CH2-, -CH2-CH2-CH2-, -CH2-CH2-CH2-CH2-,
   wobei
die Stereozentren a, b, c und d jeweils unabhängig voneinander eine(R)- oder (S)-Konfiguration aufweisen.

Für die bevorzugten Ausführungsformen der Tetrahydrofuranlignane der Formel (I) wird auf die obigen Ausführung dazu verwiesen.

Ein weiterer Aspekt der vorliegenden Erfindung betrifft ein Verfahren zum Erreichen einer physiologischen Kühlwirkung auf der Haut und/oder einer Schleimhaut durch die Anwendung von Zubereitungen, welche Tetrahydrofuranlignane der Formel (I) umfassen, vorzugsweise Aromazubereitungen.

Ein solches erfindungsgemäßes Verfahren kann zu therapeutischen oder nichttherapeutischen (z. B. kosmetischen) Zwecken durchgeführt werden und umfasst die folgenden Schritte:

Applizieren einer zum Erreichen einer physiologischen Kühlwirkung ausreichenden Menge an Tetrahydrofuranlignane der Formel (I) oder ein Extrakt davon, welche in
(i) eine pharmazeutische Zubereitung oder
(ii) eine kosmetische Zubereitung vorliegt,
auf die Haut und/oder Schleimhaut.

Ferner ist ein Gegenstand der vorliegenden Erfindung ein Verfahren zum Maskieren, Vermindern, Unterdrücken und/oder Überdecken eines unangenehmen Geschmacks, ausgewählt aus bitter, astringend und / oder den Nachgeschmack von Süßstoffen und / oder zur Verstärkung eines angenehmen Geschmacks, umfassend die Schritte:
(i) Bereitstellung von einer Zusammensetzung, welche mindestens eine Verbindung umfasst, die einen unangenehmen Geschmack in der Mundhöhle vermittelt, insbesondere in Richtung bitter, astringent und / oder den schlechten Nachgeschmack von Süßststoff,
(ii) Bereitstellung von mindestens einem Tetrahydrofuranlignane der Formel (I) oder ein Extrakt davon, oder einer erfindungsgemäßen Aromazusammensetzung, umfassend mindestens ein tetrahydrofuranlignane der Formel (I),
(iii) Mischen beider Zusammensetzungen aus Schritt a) und b),
so dass eine Zusammensetzung erhalten wird, welche dann in Zubereitungen, insbesondere im Bereich Nahrungs-, Lebens- oder Genussmittel eingesetzt werden kann.

### BEISPIELE

### Herstellbeispiele 1 bis 6

### Herstellbeispiel 1: 6-[(3S,3aR,6S,6aR)-3-(3,4-Dimethoxyphenyl)-1,3,3a,4,6,6a-hexahydro furo[3,4-c]furan-6-yl]-4-methoxy-1,3-benzodioxole (Verbindung 1)

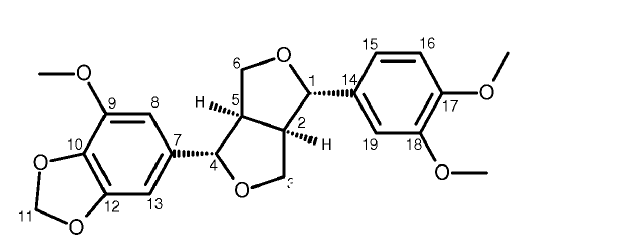

### Spektroskopische Daten:

| **Position** | **¹H NMR 400 Hz, CDCl₃** | **¹³C NMR 100 MHz, CDCl₃** |
|---|---|---|
| | ***δ*_{H}(ppm), mult (*J* in Hz)** | ***δ*_{c} (ppm)** |
| 1 | 4,74 d (4,8) | 85,7 CH |
| 2 | 3,07 m | 54,1; 54,4 CH |
| 3 | 3,89 m, 4,26 dd (6,6; 9,2) | 71,7 CH2 |
| 4 | 4,72 d (4,7) | 85,9 CH |
| 5 | 3,07 m | 54,1; 54,4 CH |
| 6 | 3,89 m, 4,25 dd (6,6; 9,2) | 71,7 CH2 |
| 7 | | 135,8 C |
| 8 | 6,55 dd (0,6; 1,5) | 105,5 CH |
| 9 | | 143,6 C |
| 10 | | 134,6 C |
| 11 | 5,96 s | 101,5 CH2 |
| 12 | | 149,1 C |
| 13 | 6,53 dd (0,5; 1,5) | 100,0 CH |
| 14 | | 133,4 C |
| 15 | 6,87 ddd (0,6; 1,9; 8,2) | 118,2 CH |
| 16 | 6,84 d (8,2) | 111,0 CH |
| 17 | | 148,6 C |
| 18 | | 149,1 C |
| 19 | 6,90 d (1,9) | 109,1 CH |
| 9 OCH3 | 3,91 s | 56,7 |
| 17 OCH3 | 3,88 s | 55,9 |
| 18 OCH3 | 3,90 s | 55,9 |

| **HR-MS(calc)** | **HR-MS** | **[*M*+H]⁺** | ***m*/*z*** | **UV λₘₐₓ(nm)** |
|---|---|---|---|---|
| 401,1595 | 401,1575 | C₂₂H₂₅O₇ | 151,0762; 165,0553 | 220 |

### Herstellbeispiel 2: (+)-Diasesartemin (4-Methoxy-6-[(1R,3aR,4S,6aR)-tetrahydro-4-(3,4,5-trimethoxyphenvl)-1H,3H-furo[3,4-c]furan-1-yl]-1,3-benzodioxol, Verbindung 2)

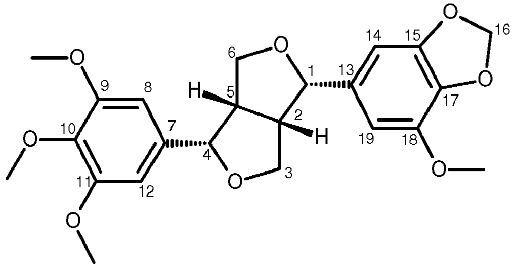

### Spektroskopische Daten:

| **Position** | **¹H NMR 400 MHz, CDCl₃** | **¹³C NMR 100 MHz, CDCl₃** |
|---|---|---|
| | ***δ*_{H} (ppm), mult (*J* in Hz)** | ***δ*_{c} (ppm)** |
| 1 | 4,88 d (5,5) | 84,1 CH |
| 2/5 | 3,17 m | 49,5; 49,5 CH |
| 3/6 | 3,56; 3,71 dd (2,4; 6,9; 9,6; 9,8), 3,57; 3,74 dd (2,1; 6,8; 9,7; 9,8) | 68,8; 68,9 CH2 |
| 4 | 4,90 d (5,5) | 84,0 CH |
| 7 | | 134,3 C |
| 8/12 | 6,59 d (0,7) | 103,2 CH |
| 9/11 | | 153,2 C |
| 10 | | 137,1 C |
| 13 | | 133,5 C |
| 14 | 6,56 dd (0,7; 1,4) | 100,6 CH |
| 15 | | 148,9 C |
| 16 | 5,98 s | 101,4 CH2 |
| 17 | | 134,6 C |
| 18 | | 143,6 C |
| 19 | 6,58 dd (0,8; 1,4) | 105,7 CH |
| 9/11 OCH3 | 3,89 s | 56,2 CH3 |
| 10 OCH3 | 3,85 s | 60,9 CH3 |
| 18 OCH3 | 3,92 s | 56,6 CH3 |

| **HR-MS(calc)** | **HR-MS** | **[*M*+H]⁺** | ***m*/*z*** | **UV λₘₐₓ (nm)** |
|---|---|---|---|---|
| 431,1700 | 431,1697 | C₂₃H₂₇O₈ | 165,0560 | 220 |

### Herstellbeispiel 3: (+)-Sesartemin (6-[(3R,3aS,6R,6aS)-6-(3,4,5-Trimethoxyphenyl)-1,3,3a,4,6,6a-hexahydrofuro[3,4-c]furan-3-yl]-4-methoxy-1,3-benzodioxol, Verbindung 3)

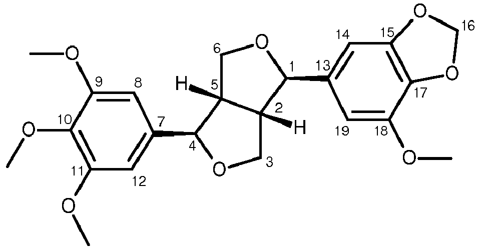

### Spektroskopische Daten:

| **Position** | **¹HHMR 400 MHz, CDCl₃** | **¹³C NMR 100 MHz, CDCl₃** |
|---|---|---|
| | ***δ*_{H} (ppm), mult (*J* in Hz)** | ***δ*_{c} (ppm)** |
| 1/4 | 4,72 d (4,3), 4,73 d (4,6) | 85,8; 86,0 CH |
| 2/5 | 3,07 m | 54,4 CH |
| 3/6 | 3,90; 3,91 dd (3,7; 3,8; 9,1; 9,2), 4,27; 4,29 dd (6,7; 6,8; 8,8; 8,9) | 71,8; 72,0 CH2 |
| 7 | | 136,7 C |
| 8/12 | 6,57 d (0,5) | 102,9 CH |
| 9/11 | | 153,4 C |
| 10 | | 137,5 C |
| 13 | | 135,8 C |
| 14 | 6,53 dd (0,6; 1,4) | 100,1 CH |
| 15 | | 149,1 C |
| 16 | 5,96 d (1,5) | 101,5 CH2 |
| 17 | | 134,7 C |
| 18 | | 143,7 C |
| 19 | 6,55 dd (0,6; 1,4) | 105,6 CH |
| 9/11 OCH3 | 3,87 s | 56,20 CH3 |
| 10 OCH3 | 3,84 s | 60,84 CH3 |
| 18 OCH3 | 3,92 s | 56,73 CH3 |

| **HR-MS (calc)** | **HR-MS** | **[*M*+H]⁺** | ***m*/*z*** | **UV λₘₐₓ (nm)** |
|---|---|---|---|---|
| 431,1700 | 431,1684 | C₂₃H₂₇O₈ | 165,0555 | 220 |

### Herstellbeispiel 4: (+)-Episesartemin B (6-[(3S,3aR,6R,6aR)-6-(3,4,5-Trimethoxyphenyl)-1,3,3a,4,6,6a-hexahydrofuro[3,4-c]furan-3-yl]-4-methoxy-1,3-benzodioxol, Verbindung 4)

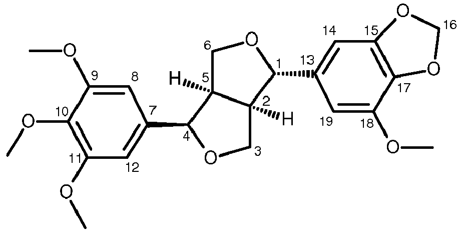

### Spektroskopische Daten:

| **Position** | **¹H MR400 MHz, CDCl₃** | **¹³C NMR 100 MHz, CDCl₃** |
|---|---|---|
| | ***δ*_{H} (ppm), mult (*J* in Hz)** | ***δ*_{c} ppm** |
| 1 | 4,41 d (7,0) | 87,7 CH |
| 2 | 2,88 dddd (1,3; 6,3; 6,8; 9,0) | 54,6 CH |
| 3/6 | 3,33 m, 3,87 m, 4,13 dd (1,2; 9,5) | 69,7; 71,0 CH2 |
| 4 | 4,85 d (5,4) | 82,1 CH |
| 5 | 3,33 m | 50,0 CH |
| 7 | | 134,0 C |
| 8/12 | 6,5 8 d (0,7) | 102,5 CH |
| 9/11 | | 153,2 C |
| 10 | | 136,8 C |
| 13 | | 135,8 C |
| 14 | 6,55 dd (0,4; 1,4) | 100,2 CH |
| 15 | | 149,0 C |
| 16 | 5,96 d (1,5) | 101,5 CH2 |
| 17 | | 134,7 C |
| 18 | | 143,6 C |
| 19 | 6,57 dd (0,4; 1,4) | 105,5 CH |
| 9/11 OCH3 | 3,88 s | 56,1 CH3 |
| 10 OCH3 | 3,85 s | 60,9 CH3 |
| 18 OCH3 | 3,91 s | 56,6 CH3 |

| **HR-MS(calc)** | **HR-MS** | **[*M*+H]⁺** | ***m*/*z*** | **UV λₘₐₓ (nm)** |
|---|---|---|---|---|
| 431,1700 | 431,1695 | C₂₃H₂₇O₈ | 165,0561; 203,0714 | 220 |

### Herstellbeispiel 5: (+)-Episesartemin A (6-[(3R,3aR,6S,6aR)-6-(3,4,5-Trimethoxyphenyl)-1,3,3a,4,6,6a-hexahydrofuro[3,4-c]furan-3-yl]-4-methoxy-1,3-benzodioxol, Verbindung 5)

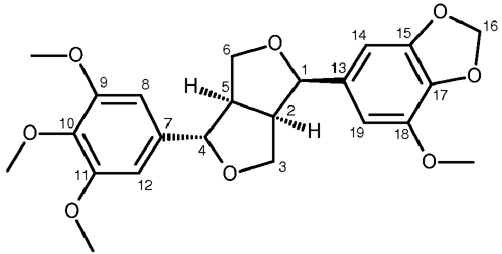

### Spektroskopische Daten:

| **Position** | **¹H NMR 400 MHz, CDCl₃** | **¹³ C NMR 100 MHz,CDCl₃** |
|---|---|---|
| | *δ*_{H} **(ppm), mult (*J* in Hz)** | ***δ*_{c} (ppm)** |
| 1 | 4,83 d (5,4) | 82,1 CH |
| 2 | 3,33 m | 50,1 CH |
| 3/6 | 3,33 m, 3,86 m, 4,14 dd (1,2; 9,5) | 69,7; 71,0 CH2 |
| 4 | 4,42d (7,1) | 87,8 CH |
| 5 | 2,91 dddd (1,4; 6,0; 7,2; 8,9) | 54,6 CH |
| 7 | | 136,8 C |
| 8/12 | 6,59 d (2,5) | 103,0 CH |
| 9/11 | | 153,4 C |
| 10 | | 137,6 C |
| 13 | | 132,9 C |
| 14 | 6,52 dd (0,8; 1,4) | 99,9 CH |
| 15 | | 148,9 C |
| 16 | 5,97 m | 101,4 CH2 |
| 17 | | 134,2 C |
| 18 | | 143,6 C |
| 19 | 6,59 d (2,5) | 105,0 CH |
| 9/11 OCH3 | 3,88 s | 56,2 CH3 |
| 10 OCH3 | 3,84 s | 60,8 CH3 |
| 18 OCH3 | 3,93 s | 56,7 CH3 |

| **HR-MS (calc)** | **HR-MS** | **[*M*+H]⁺** | ***m*/*z*** | **UV λₘₐₓ (nm)** |
|---|---|---|---|---|
| 431,1700 | 431,1670 | C₂₃H₂₇O₈ | 165,0550; 203,1701 | 225 |

### Herstellbeispiel 6: (+)-Diayangambin ((3R,3aR,6R,6aR)-3,6-Bis(3,4,5-trimethoxyphenyl)-1,3,3a,4,6,6a-hexahydrofuro[3,4-c]furan, Verbindung 6)

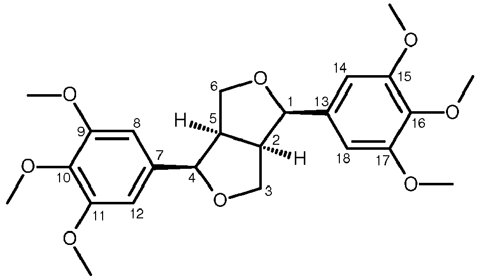

### Spektroskopische Daten:

| **Position** | **¹H NMR 400 MHz, DMSO-*d6*** | **¹³C NMR 100 MHz, DM9O-*d6*** |
|---|---|---|
| | ***δ*_{H} (ppm), mult (*J* in Hz)** | ***δ*_{c} (ppm)** |
| 1/4 | 4,86 d (4,9) | 83,1 CH |
| 2/5 | 3,30 m | 48,6 CH |
| 3/6 | 3,44; 3,50 dd (2,0; 7,0; 9,3; 9,4) | 68,2 CH2 |
| 7/13 | | 135,0 C |
| 8/12/14/18 | 6,64 s | 103,2 CH |
| 9/11/15/17 | | 152,7 C |
| 10/16 | | 136,2 C |
| 9/11/15/17 | | |
| OCH3 | 3,78 s | 55,8 CH3 |
| 10/16 OCH3 | 3,67 s | 60,0 CH3 |

| **HR-MS (calc)** | **HR-MS** | **[*M*+H]⁺** | ***m*/*z*** | **UV λₘₐₓ (nm)** |
|---|---|---|---|---|
| 447,2013 | 447,2002 | C₂₄H₃₁O₈ | 181,0868; 204,0788; 219,1013 | 210,270 |

### Anwendungsbeispiele 7 bis 8

### Anwendungsbeispiel 7: Aroma- und Kühlwirkung der Verbindungen 4 und 6

Die beispielhaften Verbindungen 4 und 6 wurden auf ihre sensorischen Eigenschaften, insbesondere ihre Kühlwirkung getestet. Dafür wurden sie jeweils in einer bestimmten Endkonzentration in Wasser gelöst und in einer Runde von 5 bis 10 Experten bewertet. Die sensorischen Eindrücke, insbesondere die trigeminalen Effekte wurden notiert.

| **Probe** | **Konzentration (ppm)** | **Sensoriche Beschreibung** |
|---|---|---|
| (+)-Episesartemin B (**Verbindung 4**) | 10 | schwach brennend/wärmend |
| 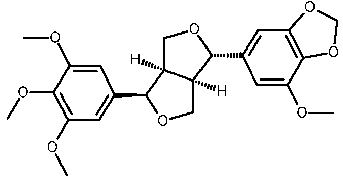 | 50 | schwach bitter, schwach brennend, betäubend, kühlend |
| (+)-Diayangambin (**Verbindung 6**) | 10 | tingling (verzögert), mundwässernd, frisch, kühlend |
| 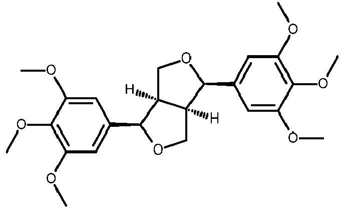 | 50 | tingling (verzögert), mundwässernd, kühlend, irritierend |

### Anwendungsbeispiel 8: Aromazusammensetzung A-F

Durch Mischung der Komponenten in der Tabelle 1 werden stark kühlende Aromamischungen erhalten.

| **Inhaltsstoff** | **Anteil in Gew-%** | | | | | |
|---|---|---|---|---|---|---|
| | **A** | **B** | **C** | **D** | **E** | **F** |
| (+)-Episesartemin B (Verbindung 4) | - | 20 | - | 10 | - | 20 |
| (+)-Diayangambin (Verbindung 6) | 18 | 18 | 18 | 15 | 25 | 20 |
| L-Menthyllactat (Frescolat ML, Symrise) | - | 10 | 30 | 65 | 65 | - |
| L-Menthol | 2 | - | - | - | - | - |
| Natürliches Zitronenaroma | - | 2 | 2 | - | - | - |
| Pfefferminzöl | - | - | - | 10 | - | - |
| Natürliches Pfirsicharoma | - | - | - | - | - | 10 |
| Lösung aus 10 Gew.-% Pellitorin in Propylenglycol/Pfefferminzöl 1:1 | - | - | - | - | 10 | - |
| Propylenglycol | Ad 100 | Ad 100 | Ad 100 | Ad 100 | Ad 100 | Ad 100 |

### Verwendungsbeispiele 9-14:

### Verwendungsbeispiel 9: Zahnpasta

Die Inhaltsstoffe der Teile A und B wurden jeweils für sich vorgemischt und zusammen unter Vakuum bei 25 - 30°C jeweils 30 min lang gut verrührt. Teil C wurde vorgemischt und zu A und B gegeben; D wurde hinzugefügt und die Mischung unter Vakuum bei 25 - 30°C weitere 30 min gut verrührt. Nach Entspannung war die Zahnpasta fertig und konnte abgefüllt werden.

| **Teil** | **Inhaltsstoff** | **Einsatz in Gew.-%** |
|---|---|---|
| A | demineralisiertes Wasser | 22,00 |
| | Sorbitol (70%) | 45,00 |
| | Solbrol^{®} M, Natriumsalz (Bayer AG, p-Hydroxybenzoesäurealkylester) | 0,15 |
| | Trinatriumphosphat | 0,10 |
| | Saccharin, 450 fach | 0,20 |
| | Natriummonofluorphosphat | 1,12 |
| | Polyethylenglycol 1500 | 5,00 |
| B | Sident 9 (abrasives Siliciumdioxid) | 10,00 |
| | Sident 22 S (verdickendes Siliciumdioxid) | 8,00 |
| | Natriumcarboxymethylcellulose | 0,90 |
| | Titandioxid | 0,50 |
| C | demineralisertes Wasser | 4,53 |
| | Natriumlaurylsulfat | 1,50 |
| D | Aromazusammensetzung D aus Beispiel 8 | 1 |

### Verwendungsbeispiel 10: Zuckerfreier Kaugummi

Teile A bis D wurden gemischt und intensiv geknetet. Die Rohmasse wurde z.B. in Form von dünnen Streifen zu verzehrsfertigen Kaugummis verarbeitet.

| **Teil** | **Inhaltsstoff** | **Einsatz in Gew.-%** |
|---|---|---|
| A | Kaugummibase, Company "Jagum T" | 30,00 |
| B | Sorbit, pulverisiert | 39,00 |
| | Isomalt^{®} (Palatinit GmbH) | 9,50 |
| | Xylit | 2,00 |
| | Mannit | 3,00 |
| | Aspartam^{®} | 0,10 |
| | Acesulfam^{®} K | 0,10 |
| | Emulgum^{®} (Colloides Naturels, Inc.) | 0,30 |
| C | Sorbitol, 70% | 14,00 |
| | Glycerin | 1,00 |
| D | Spearmint-Pfefferminz-Eucalyptus-Aroma, enthaltend 10 Gew.-% (+)-Diayangambin (Verbindung 6) | 1 |

### Verwendungsbeispiel 11: Mundwasser

Die Inhaltsstoffe der Teile A und B wurden jeweils für sich gemischt. Teil B wurde langsam in Teil A eingerührt, bis die Mischung homogen war.

| **Teil** | **Inhaltsstoff** | **Einsatz in Gew. -%** |
|---|---|---|
| A | Ethanol | 10,00 |
| | Cremophor^{®} CO 40 (BASF, Detergenz) | 1,00 |
| | Benzoesäure | 0,12 |
| | Pfefferminz-Zitronenmelisse-Aroma, enthaltend 0,4 Gew.-% Pellitorin und 10 Gew.-% (+)-Diayangambin (Verbindung 6) | 0,25 |
| B | demineralisiertes Wasser | 83,46 |
| | Sorbitol, 70% | 5,00 |
| | Natriumsaccharin 450 | 0,07 |
| | L-Blue 5000 e.c., 1% in Wasser (Farbstoff) | 0,10 |

### Verwenudngsbeispiel 12: Halsbonbons mit flüssig-viskoser Kernfüllung (centre-filled hard candy)

In Anlehnung an die in US 6,432,441 (dort Beispiel 1) sowie die in US 5,458,894 bzw. US 5,002,791 beschriebenen Verfahren wurden Bonbons mit flüssig-viskosem Kern hergestellt. Beide Mischungen A und B wurden separat zu Basen für Hülle (Mischung A) bzw. Kern (Mischung B) verarbeitet.

| | **G (Gew.-%)** | **H (Gew.-%)** |
|---|---|---|
| **Mischung A (Hülle)** (**80**% **der Bonbons)** | | |
| Zucker(Saccharose) | 58,12 | 49,37 |
| Glucosesirup (Feststoffgehalt 80%) | 41,51 | 49,37 |
| Aromazusammensetzung E aus Beispiel 8 | 0,17 | 0,25 |
| I-Menthol | 0,10 | - |
| Citronenöl | 0,10 | 0,10 |
| Citronensäure | - | 0,91 |
| Total: | 100 | 100 |

| **Mischung B (Kern) (20% der Bonbons)** | | |
|---|---|---|
| High Fructose Maissirup (Gehalt an festen Zuckern 85%, knapp 15% Wasser) | 84,38 | 84,36 |
| Glycerin | 15,0 | 15,0 |
| Lecithin | 0,02 | 0,02 |
| Zimtöl | - | 0,32 |
| Spearmintöl | 0,28 | - |
| Capsaicin | 0,05 | - |
| Vanillylalkohol-n-butylether | - | 0,10 |
| Roter Farbstoff, als 5%ige wässrige Lösung | 0,20 | 0,20 |
| Vanillin | 0,07 | - |
| Total | 100 | 100 |

### Verwendungsbeispiel 13: Kaugummi

Die Kaugummibase K2 bestand aus folgenden Zutaten: 28,5% Terpenharz, 33,9% Polyvinylacetat (MW = 14,000), 16,25% hydriertes Pflanzenöl, 5,5% Mono- und Diglyceride, 0,5% Polyisobuten (MW 75,000), 2,0% Butyl Rubber (Isobuten-Isopren-Copolymer), 4,6% amorphes Siliciumdioxid (Wassergehalt ca. 2,5%), 0,05% Antioxidans tert.-Butylhydroxytoluol (BHT), 0,2% Lecitihin, und 8,5% Calciumcarbonat. Die Herstellung der Kaugummibase K2 und der Kaugummis erfolgte analog zu US 6,986,907.

| | **I (Gew.-%)** | **J (Gew.-%)** | **K (Gew.-%)** |
|---|---|---|---|
| Kaugummibase K2 | 25,30 | 27,30 | 26,30 |
| Sorbitol | 61,48 | 59,48 | 61,80 |
| Glycerin | 2,40 | 2,40 | 2,40 |
| Lecithin | 7,00 | 7,00 | 7,00 |
| Aspartam | 0,14 | 0,14 | 0,14 |
| Verkapseltes Aspartam | 0,68 | 0,68 | 0,68 |
| Menthol, sprühgetrocknet | 0,50 | - | - |
| Kirscharoma, sprühgetrocknet | - | 1,20 | - |
| Aromazusammensetzung D aus Beispiel 8, sprühgetrocknet | 1,50 | 1,80 | - |
| Aromazusammensetzung C aus Beispiel 8 | 1,00 | - | 1,68 |

Die Kaugummis der Rezeptur I und J wurden als Streifen, die der Rezeptur K als Pellets ausgeformt.

### Verwendungsbeispiel 14: Kaubonbon

Herstellungshinweise:
a) Gelatine mit Wasser (1,8 fache Menge der Gelatine) bei 70°C 2 Stunden quellen lassen;
b) Zucker, Sirup, Wasser, Fett und Lecithin auf 123°C kochen;
c) Gelatinelösung langsam mit dem Kochansatz vermischen;
d) Aroma aus Beispiel 8 und optional Farbe unterrühren;
e) resultierende Masse auf einem Kühltisch auf ca. 70°C temperieren, danach Fondant zugeben und auf einer Ziehmaschine ca. 3 Minuten belüften;
f) Kaubonbonmasse anschließend schneiden und verpacken.

Beim Verzehr der Kaubonbons wird während des Kauens ein frischer, kühlender Himbeergeschmack wahrgenommen.

| | | Gew-% |
|---|---|---|
| Wasser | | 7,80 |
| Zucker | Raffinade C4 | 42,10 |
| Glucose Sirup | Dextrose 40 | 37,30 |
| gehärtetes Pflanzenfett | Schmelzpunkt 32-36°C | 6,60 |
| Lecithin | Emulgator (Sojalecithin) | 0,30 |
| Gelatine | Schweinegelatine | 0,80 |
| Fondant | Typ-S30 | 4,80 |
| Himbeeraroma | | 0,22 |
| Aromazusammensetzung C aus Beispiel 8 | | 0,08 |

### Anwendungsbeispiele 15 bis 16

### Anwendungsbeispiel 15: Herstellung eines Extrudat

### Herstellungshinweis (siehe auch WO 03/092412):

Alle Bestandteile wurden gemischt und per Einpunktdosierung in einen Doppelschneckenextruder gefördert. Die Extrusionstemperaturen lagen zwischen 100 und 120°C, der spezifische Energieeintrag lag bei 0,2 kWh/kg. Die aus der mit 1 mm Bohrungen versehene Düsenplatte des Extruders austretenden Stränge wurden unmittelbar nach Austritt aus den Düsen durch rotierende Messer auf Partikel mit ca. 1 mm Durchmesser geschnitten.

| | | |
|---|---|---|
| Glukosesirup, sprühgetrocknet (DE-Wert: 31-34) | Glucidex IT33W (Firma Roquette) | 62,0 % |
| Maltodextrin (DE-Wert: 17-20) | (Firma Cerestar) | 28,4 % |
| Emulgator Monomuls | Emulgator auf der Basis von gehärtetem Palmöl; Schmelzpunkt: 64°C, (Firma Grünau) | 1,8 % |
| Dextrosemonohydrat (DE-Wert: 99,5) | Dextrose, kristallwasserhaltig (Firma Cerestar) | 1,8 % |
| Wasser | | 2,0% |
| Orangen-Vanillearoma | | 3,2 % |
| Aromazusammensetzung D aus Beispiel 8 | | 0,8 % |

### Anwendungsbeispiel 16: Herstellung von Granulaten mittels Wirbelschicht

In einer Granulierapparatur des in EP 163 836 dargestellten Typs (mit den folgenden Merkmalen: Durchmesser Anströmboden: 225 mm, Sprühdüse: Zweistoffdüse; Sichtender Austrag: Zick-Zack-Sichter; Filter: internes Schlauchfilter) wird eine Lösung bestehend aus 44 Gew.-% Wasser, 8 Gew.% Citronenaroma, 3 Gew.-% Aromazusammensetzung D aus Beispiel 8, 13 Gew.-% Gummi arabicum und 32 Gew.-% hydrolysierter Stärke (Maltodextrin DE 15-19) sowie etwas grünem Farbstoff granuliert. Die Lösung wird bei einer Temperatur von 32°C in den Wirbelschichtgranulator gesprüht. Zur Fluidisierung des Bettinhaltes wird Stickstoff in einer Menge von 140 kg/h eingeblasen. Die Eintrittstemperatur des Fluidisiergases beträgt 140°C. Die Temperatur des Abgases beträgt 76°C. Als Sichtgas wird ebenfalls Stickstoff in einer Menge von 15 kg/h mit einer Temperatur von 50°C zugeführt. Der Inhalt des Wirbelbettes beträgt ca. 500 g. Die Granulierleistung beträgt ca. 2,5 kg pro Stunde. Man erhält ein frei fließendes Granulat mit einem mittleren Teilchendurchmesser von 360 Mikrometern. Die Granulate sind rund und weisen eine glatte Oberfläche auf. Aufgrund des konstanten Druckverlustes des Filters und des ebenfalls konstant bleibenden Bettinhalts ist von stationären Bedingungen hinsichtlich des Granulationsprozesses auszugehen.

### Verwendungsbeispiele 17-19:

### Verwendungsbeispiel 17: Teebeutel mit grünem bzw. schwarzem Tee

Jeweils 800 g Grüntee (Typ Sencha) wurden einmal mit 33 g der Extrudate aus Beispiel 15 und einmal mit 30 g Granulaten aus Beispiel 16 gemischt, portioniert und anschließend in Teebeutel abgefüllt.
Jeweils 800 g schwarzer Tee (Blattgrad Fannings) wurden einmal mit 33 g der Extrudate aus Beispiel 15 und einmal mit 30 g Granulaten aus Beispiel 16 gemischt, portioniert und anschließend in Teebeutel abgefüllt.

### Verwendungsbeispiel 18: Grüntee-Zubereitungen

Der Grünteeextrakt, Säure und Süßstoffmischung sowie die Aromakompositionen wurden in 80 °C heißem Wasser gelöst und in Flaschen abgefüllt.

| **Inhaltsstoff** | **Einsatz in Gew.-%** | | | |
|---|---|---|---|---|
| | **L** | **M** | **N** | **O** |
| Grüntee-Extrakt, ca. 16% Catechingehalt | 0,25 | 0,25 | 0,5 | 0,25 |
| Aromazusammensetzung B aus Beispiel 8 | 0,1 | - | - | 0,1 |
| Aromazusammensetzung C aus Beispiel 8 | - | 0,1 | - | - |
| Aromazusammensetzung F aus Beispiel 8 | - | - | 0,1 | - |
| Zucker | 5 | 2,5 | 2,5 | - |
| Rebaudiosid A | - | 0,025 | 0,025 | - |
| Süßstoffmischung (Aspartam, Sucralose 1:1) | - | - | - | 0,01 |
| Äpfelsäure, Citronensäure | 0,1 | 0,1 | 0,1 | 0,1 |
| Demineralisiertes Wasser | ad 100 | ad 100 | ad 100 | ad 100 |

### Verwendungsbeispiel 19: Eistee (kalorienreduziert)

| **Inhaltsstoff** | **Einsatz in Gew.-%** | | | | |
|---|---|---|---|---|---|
| | **P** | **Q** | **R** | **S** | **T** |
| Grüntee Extrakt, Catechingehalt10 Gew.% | 1,4 | 1,2 | - | - | 0,7 |
| Schwarztee Extrakt | - | - | 1,4 | 1,4 | 0,7 |
| Natürliches Aroma Typ Zitrone | 0,65 | - | 0,65 | - | - |
| Aroma Typ Pfirsich | - | 0,65 | - | 0,65 | - |
| Sucrose | 3,45 | 3,45 | 6 | 3,45 | 2,5 |
| Aspartam/Sucralose 1:1-Mischung | 0,1 | - | - | 0,1 | - |
| Rebaudiosid A Gew.-95% | - | 0,02 | - | - | 0,04 |
| Zitronensäure (kristallin) | 1,2 | 1,2 | 1,2 | 1,2 | 1,2 |
| Ascorbinsäure | 0,2 | 0,2 | 0,2 | 0,2 | 0,2 |
| Aromazusammensetzung C aus Beispiel 8 | 0,05 | - | 0,05 | - | - |
| Aromazusammensetzung D aus Beispiel 8 | - | - | - | - | 0,05 |
| Aromazusammensetzung F aus Beispiel 8 | - | 0.05 | - | 0,05 | - |
| Wasser | Ad 100 | Ad 100 | Ad 100 | Ad 100 | Ad 100 |

Die in den Anwendungsbeispielen gefundenen Effekte lassen sich - gegebenenfalls durch vom Fachmann unschwer vorzunehmende Modifikationen - auf alle Produkte der jeweiligen Produktgruppe, d.h. insbesondere auf Zahnpasten, Kaugummis, Mundwässer, Halsbonbons, Gelatinekapseln, Kaubonbons und Tee in Beuteln übertragen. Dabei ist es für den Fachmann aufgrund der vorliegenden Beschreibung unschwer erkennbar, dass ohne großen Aufwand die erfindungsgemäßen Verbindungen, Gemische und Mischungen - eventuell unter geringfügigen Modifikationen - untereinander austauschbar sind. Das bedeutet, dass die in den Produkten der Anwendungsbeispiele verwendete erfindungsgemäße Verbindung als Platzhalter auch für die anderen erfindungsgemäßen Verbindungen, Mischungen und Gemische aufgefasst werden muss. Auch die Konzentration der verwendeten erfindungsgemäßen Verbindung, Mischung oder des verwendeten Gemisches ist für den Fachmann leicht erkenntlich variierbar. Außerdem sind die produktspezifischen weiteren Bestandteile in dem jeweiligen Anwendungsbeispiel für den Fachmann leicht nachvollziehbar ebenfalls gegen weitere produkttypische Bestandteile austauschbar bzw. durch solche ergänzbar. Eine Vielzahl solcher produktspezifischer Bestandteile ist in der oben stehenden Beschreibung offenbart.

## Patentansprüche

1. Verwendung von Tetrahydrofuranlignane der allgemeinen Formel (I): 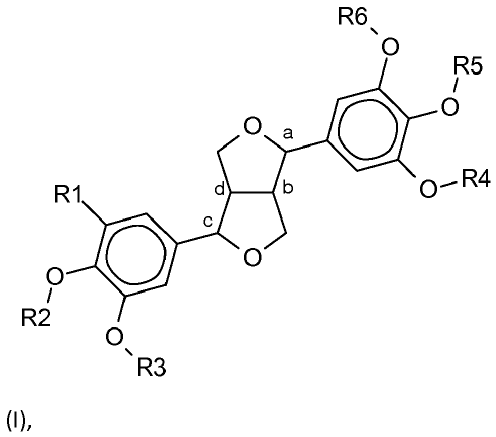 wobei R1, R2, R3, R4, R5 und R6 unabhängig voneinander ausgewählt sind aus der Gruppe bestehend aus: Wasserstoff, C₁-C₃-Alkyl, und/oder R2 und R3 jeweils zusammen und / oder R5 und R6 jeweils zusammen einen carbocyclischen Ring ausbilden können, ausgewählt aus der Gruppe bestehend aus:
-CH₂-, -CH₂-CH₂-, -CH₂-CH₂-CH₂-, -CH₂-CH₂-CH₂-CH₂-,
wobei die Stereozentren a, b, c und d jeweils unabhängig voneinander eine(R)- oder (S)-Konfiguration aufweisen,
oder ein Extrakt davon,
(i) als Kühlwirkstoffe und / oder
(ii) als Geschmacksmodifizierer und / oder
(iii) als Aromastoffe und / oder als geschmacksmodulierende Aromastoffe zur
(a) Maskierung und/oder Verminderung und/oder Unterdrückung und/oder Überdeckung eines unangenehmen Geschmacks, ausgewählt aus bitter, astringend und / oder den Nachgeschmack von Süßstoffen , und / oder
(b) Verstärkung eines angenehmen Geschmacks.

2. Verwendung nach Anspruch 1, wobei
R1 Wasserstoff oder C₁-C₃-Alkyl ist, und
R2, R3, R4, R5, und R6 unabhängig voneinander ein C₁-C₃-Alkyl darstellen, und/oder R2 und R3 jeweils zusammen und/oder
R5 und R6 jeweils zusammeneinen carbocyclischen Ring ausbilden können, ausgewählt aus der Gruppe bestehend aus: -CH₂-, -CH₂-CH₂-, -CH₂-CH₂-CH₂-, -CH₂-CH₂-CH₂-CH₂-wobei die Stereozentren a, b, c und d jeweils unabhängig voneinander eine(R)- oder (S)-Konfiguration aufweisen.

3. Verwendung nach den Ansprüchen 1 und/oder 2, wobei
R1 Wasserstoff oder C₁-C₃-Alkyl ist, und
R2, R3, R4, R5, und R6 unabhängig voneinander ein C₁-C₃-Alkyl darstellen, und/oder R5 und R6 jeweils zusammen einen carbocyclischen Ring ausbilden können, ausgewählt aus der Gruppe bestehend aus: -CH₂-, -CH₂-CH₂-, -CH₂-CH₂-CH₂-, -CH₂-CH₂-CH₂-CH₂-,
wobei die Stereozentren a, b, c und d jeweils unabhängig voneinander eine (R)- oder (S)-Konfiguration aufweisen.

4. Verwendung nach mindestens einem der Ansprüche 1 bis 3, wobei der carbocyclischer Ring, welcher durch R2 und R3 zusammen und/oder R5 und R6 zusammen ausgebildet wird, jeweils unabhängig voneinander ein Dioxolanring darstellen kann.

5. Verwendung nach einem der Ansprüche 1 bis 4, wobei die C1-C3-Alkygruppe eine Methylgruppe ist.

6. Verwendung nach mindestens einem der Ansprüche 1 bis 5, wobei die Tetrahydrofuranlignane der allgemeinen Formel (I) ausgewählt sind aus der Gruppe bestehend aus:
6-[((3S,3aR,6S,6aR)-3-(3,4-Dimethoxyphenyl)-1,3,3a,4,6,6a-hexahydrofuro[3,4-c]furan-6-yl]-4-methoxy-1,3-benzodioxole (Verbindung 1)
(+)-Diasesartemin (4-Methoxy-6-[(1R,3aR,4S,6aR)-tetrahydro-4-(3,4,5-trimethoxyphenyl)-1H,3H-furo[3,4-c]furan-1-yl]-1,3-benzodioxol)(Verbindung 2)
(+)-Sesartemin (6-[(3R,3aS,6R,6aS)-6-(3,4,5-Trimethoxyphenyl)-1,3,3a,4,6,6a-hexahydrofuro[3,4-c]furan-3-yl]-4-methoxy-1,3-benzodioxol)(Verbindung 3)
(+)-Episesartemin B (6-[(3S,3aR,6R,6aR)-6-(3,4,5-Trimethoxyphenyl)-1,3,3a,4,6,6a-hexahydrofuro[3,4-c]furan-3-yl]-4-methoxy-1,3-benzodioxol)(Verbindung 4)
(+)-Episesartemin A (6-[(3R,3aR,6S,6aR)-6-(3,4,5-Trimethoxyphenyl)-1,3,3a,4,6,6a-hexahydrofuro[3,4-c]furan-3-yl]-4-methoxy-1,3-benzodioxol)(Verbindung 5)
(+)-Diayangambin ((3R,3aR,6R,6aR)-3,6-Bis(3,4,5-trimethoxyphenyl)-1,3,3a,4,6,6a-hexahydrofuro[3,4-c]furan)(Verbindung 6).

7. Verwendung nach mindestens einem der Ansprüche 1 bis 6 zur physiologischen Kühlung der Haut oder Schleimhaut.

8. Aromazusammensetzung, umfassend mindestens ein Tetrahydrofuranlignan der allgemeinen Formel (I), oder ein Extrakt davon 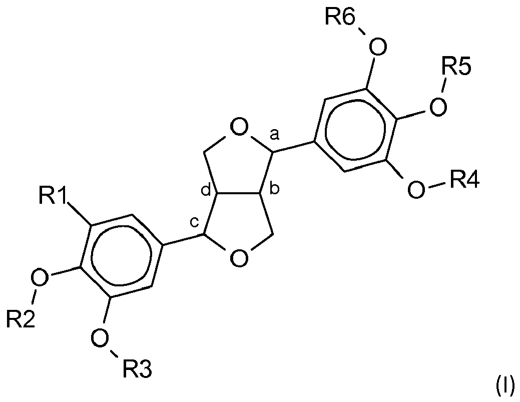 wobei
R1, R2, R3, R4, R5 und R6 unabhängig voneinander ausgewählt sind aus der Gruppe bestehend aus: Wasserstoff, C₁-C₃-Alkyl, und/oder
R2 und R3 jeweils zusammen und/oder
R5 und R6 jeweils zusammen einen carbocyclischen Ring ausbilden können, ausgewählt aus der Gruppe bestehend aus: -CH₂-, -CH₂-CH₂-, -CH₂-CH₂-CH₂-, -CH₂-CH₂-CH₂-CH₂-,
wobei die Stereozentren a, b, c und d jeweils unabhängig voneinander eine (R)- oder (S)-Konfiguration aufweisen.

9. Aromazusammensetzung nach Anspruch 8, wobei
R1 Wasserstoff oder C₁-C₃-Alkyl ist, und
R2, R3, R4, R5, und R6 unabhängig voneinander ein C1-C3Alkyl darstellen, und/oder
R2 und R3 jeweils zusammen und/oder
R5 und R6 jeweils zusammen einen carbocyclischen Ring ausbilden können, ausgewählt aus der Gruppe bestehend aus: -CH₂-, -CH₂-CH₂-, -CH₂-CH₂-CH₂-, -CH₂-CH₂-CH₂-CH₂-,
wobei die Stereozentren a, b, c und d jeweils unabhängig voneinander eine(R)- oder (S)-Konfiguration aufweisen.

10. Aromazusammensetzung nach den Ansprüchen 8 und/oder 9, wobei
R1 Wasserstoff oder C₁-C₃-Alkyl ist, und
R2, R3, R4, R5, und R6 unabhängig voneinander ein C1-C3Alkyl darstellen, und/oder
R5 und R6 jeweils zusammen einen carbocyclischen Ring ausbilden können, ausgewählt aus der Gruppe bestehend aus: -CH₂-. -CH₂-CH₂-, -CH₂-CH₂-CH₂-, -CH₂-CH₂-CH₂-CH₂-,
wobei die Stereozentren a, b, c und d jeweils unabhängig voneinander eine (R)- oder (S)-Konfiguration aufweisen.

11. Aromazusammensetzung nach mindestens einem der Ansprüche 8 bis 10, wobei der carbocyclischer Ring, welcher durch R2 und R3 zusammen und/oder R5 undR6 zusammen ausgebildet wird, jeweils unabhängig voneinander ein Dioxolanring darstellen kann und wobei die C₁-C₃-Alkylgruppe eine Methylgruppe ist.

12. Aromazusammensetzung nach mindestens einem der Ansprüche 8 bis 11, wobei die Tetrahydrofuranlignane der allgemeinen Formel (I) ausgewählt sind aus der Gruppe bestehend aus:
6-[((3S,3aR,6S,6aR)-3-(3,4-Dimethoxyphenyl)-1,3,3a,4,6,6a-hexahydrofuro[3,4-c]furan-6-yl]-4-methoxy-1,3-benzodioxole (Verbindung 1)
(+)-Diasesartemin (4-Methoxy-6-[(1R,3aR,4S,6aR)-tetrahydro-4-(3,4,5-trimethoxyphenyl)-1H,3H-furo[3,4-c]furan-1-yl]-1,3-benzodioxol)(Verbindung 2)
(+)-Sesartemin (6-[(3R,3aS,6R,6aS)-6-(3,4,5-Trimethoxyphenyl)-1,3,3a,4,6,6a-hexahydrofuro[3,4-c]furan-3-yl]-4-methoxy-1,3-benzodioxol)(Verbindung 3)
(+)-Episesartemin B (6-[(3S,3aR,6R,6aR)-6-(3,4,5-Trimethoxyphenyl)-1,3,3a,4,6,6a-hexahydrofuro[3,4-c]furan-3-yl]-4-methoxy-1,3-benzodioxol)(Verbindung 4)
(+)-Episesartemin A (6-[(3R,3aR,6S,6aR)-6-(3,4,5-Trimethoxyphenyl)-1,3,3a,4,6,6a-hexahydrofuro[3,4-c]furan-3-yl]-4-methoxy-1,3-benzodioxol)(Verbindung 5)
(+)-Diayangambin ((3R,3aR,6R,6aR)-3,6-Bis(3,4,5-trimethoxyphenyl)-1,3,3a,4,6,6a-hexahydrofuro[3,4-c]furan)(Verbindung 6).

13. Pharmazeutische oder kosmetische Zubereitung, umfassend
(i) mindestens ein Tetrahydrofuranlignane der allgemeinen Formel (I) oder ein Extrakt davon oder
(ii) eine Aromazusammensetzung nach einem der Ansprüche 8 bis 12,
(iii) mindestens ein pharmazeutisches akzeptables Trägermaterial, oder
(iv) mindestens ein kosmetisches akzeptables Trägermaterial.

14. Verfahren zum Maskieren, Vermindern, Unterdrücken und/oder Überdecken eines unangenehmen Geschmacks, ausgewählt aus bitter, astringend und / oder den Nachgeschmack von Süßstoffen und / oder zur Verstärkung eines angenehmen Geschmacks, umfassen die Schritte:
(i) Bereitstellung von einer Zusammensetzung, welche mindestens eine Verbindung umfasst, die einen unangenehmen Geschmack in der Mundhöhle vermittelt, insbesondere in Richtung bitter, astringent und / oder den schlechten Nachgeschmack von Süßstoff,
(ii) Bereitstellung von mindestens einem Tetrahydrofuranlignane der Formel (I) oder ein Extrakt davon, oder einer Aromazusammensetzung nach einem der Ansprüche 8 bis 12,
(iii) Mischen beider Zusammensetzungen aus Schritt a) und b).

15. Tetrahydrofuranlignane der allgemeinen Formel (I) oder ein Extrakt davon als Medikament, das bevorzugt als Kühlwirkstoff eingesetzt wird: 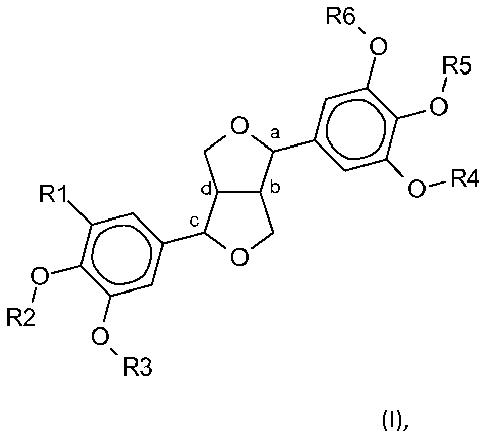 wobei
R1, R2, R3, R4, R5 und R6 unabhängig voneinander ausgewählt sind aus der Gruppe bestehend aus: Wasserstoff, C₁-C₃-Alkyl, und/oder
R2 und R3 jeweils zusammen und / oder
R5 und R6 jeweils zusammen einen carbocyclischen Ring ausbilden können, ausgewählt aus der Gruppe bestehend aus -CH₂-, -CH₂-CH₂-, -CH₂-CH₂-CH₂-, -CH₂-CH₂-CH₂-CH₂-,
wobei die Stereozentren a, b, c und d jeweils unabhängig voneinander eine (R)- oder (S)-Konfiguration aufweisen.
